(19) Europäisches Patentamt
European Patent Office
Office européen des brevets

(11) EP 0 476 457 B1

(12) **EUROPEAN PATENT SPECIFICATION**

(45) Date of publication and mention of the grant of the patent:
**04.12.1996 Bulletin 1996/49**

(51) Int Cl.[6]: **C07D 417/04**, C07D 417/14, C12Q 1/00, C12Q 1/26, G01N 33/52, A61K 31/425

(21) Application number: **91115074.6**

(22) Date of filing: **06.09.1991**

(54) **2-Benzothiazolyl tetrazolium salt indicators**

2-Benzothiazolyl-tetrazolium-Salze als Indikatoren

Sels de 2-benzothiazolyl-tétrazolium comme indicateurs

(84) Designated Contracting States:
**AT BE CH DE DK ES FR GB GR IT LI LU NL SE**

(30) Priority: **19.09.1990 US 584902**

(43) Date of publication of application:
**25.03.1992 Bulletin 1992/13**

(73) Proprietor: **Bayer Corporation**
**Pittsburgh, PA 15219-2502 (US)**

(72) Inventors:
- **Hatch, Robert P.**
  **Elkhart, IN 46515 (US)**
- **Lin, Nan-Horng**
  **Mundelein, IL 60060 (US)**
- **Ruetten, Scott**
  **Granger, IN 46530 (US)**

(74) Representative: **Dänner, Klaus, Dr. et al**
**Bayer AG**
**Konzernzentrale RP**
**Patente Konzern**
**51368 Leverkusen (DE)**

(56) References cited:
**WO-A-90/12318** **DE-A- 2 147 466**

- **CHEMICAL ABSTRACTS, vol. 104, no. 23, 9 June 1986, Columbus, Ohio, US; abstract no. 203469Y, page 407 ;**
- **CHEMISTRY OF HETEROCYCLIC COMPOUNDS (KHIMIYA GETEROTSIKLICHESKIKH SOEDINENII) vol. 7, no. 6, June 1971, pages 776 - 780; G.N. LIPUNOVA ET AL.: 'Studies in the benz- and naphthazole series ...'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

BACKGROUND OF THE INVENTION

The present invention relates to chromogenic tetrazolium salt indicator compounds useful in the determination of reducing substances, particularly nicotinamide adenine dinucleotide (NADH).

Tetrazolium salts are well known as chromogenic indicators responsive to reducing substances. Upon reduction, tetrazolium salts are converted into formazan dye products. These indicators have found use in a wide variety of fields, particularly the medical diagnostic field where they have been applied to, among others, cell staining and the determination of analytes in body fluids such as urine, milk, serum, and plasma. Commonly, the determination of body fluid analytes involves an NAD-dependent enzymatic reaction in which NADH is formed as a function of the amount of analyte present in the sample tested. The amount of NADH generated can then be determined by the reductive conversion of an appropriate tetrazolium salt indicator to its formazan dye product.

Within the field of medical diagnostic tests, tetrazolium salt indicators are useful in a variety of different product types. One particular type is the reagent strip. This product is a solid state device comprising a paper or other porous carrier matrix which is impregnated or otherwise incorporated with chemical reagents responsive to a particular analyte, for example, glucose or cholesterol. The incorporated reagent system includes a chromogenic indicator which develops color, or changes color, as a function of the amount of analyte in a sample applied to the matrix. The resulting colorimetric response can be observed visually to give qualitative or semi-quantitative readings. Quantitative results can be obtained by reading the reflectance of the matrix surface at one or more defined wavelengths with an appropriate instrument (reflectance meter).

There is a recognized need to develop tetrazolium indicators having strong absorbance at wavelengths longer than the absorbances of major interferants that can be present in the test sample. For instance, interference from hemoglobin coloration is a particular concern where the sample is whole blood. Indicators having significant absorption above about 640 nm are required in order to substantially overcome hemoglobin interference. The commonly used tetrazolium salt indicators are 2-(4-iodophenyl)-3-(4-nitrophenyl)-5-phenyltetrazolium chloride (INT), 3-(4,5-dimethylthiazol-2-yl)-2,5-diphenyltetrazolium chloride (MTT), and 2,2',5,5'-tetraphenyl-3,3'-(3,3'-dimethoxy-4,4'-biphenylene) ditetrazolium chloride (NBT). These compounds show maximum absorption (UVmax) in the range of 465 - 605 nm.

Another shortcoming of the conventionally used prior art tetrazolium salt indicators relates to the evolution of the instrumentation used to measure their colorimetric response. Rapid advancements are being made in developing smaller, less expensive reflectance meters. One of the more costly components of such meters is the optical system which comprises a light source, a filter or other spectral element for selecting or limiting the wavelength of incident or reflect light, and a sensor. Significant cost savings could be realized by eliminating or combining functions of the optical system elements or by using less expensive components, e.g., LEDs as illuminating light sources. However, commercially available LEDs emit light having a center wavelength that can vary significantly due to manufacturing variances and temperature dependence. The conventionally used tetrazolium salt indicators INT, MTT, and NBT have reflectance spectra which are strongly sloped in the region above their UVmax. Accordingly, without individually calibrating both each instrument, to account for manufacturing variability in the LED, and each test run, to account for variance due to temperature, large errors can be introduced to the assay result.

The following are representative of the prior art teachings concerning the use of various tetrazolium salts in colorimetric analysis. Tanaka et al, Japanese Kokai Tokkyo Koho JP 61000084 (Chem. Abst. 104:203469y) describes the detection of glucose using a formazan chelate obtained by the reduction of 2-(2-benzothiazolyl)-3-(carboxyphenyl) - 5-phenyl-2H-tetrazolium halide in the presence of nickel (II). Limbach et al, German DE 3,247,894 (Chem. Abst. 101: 125929v) relates to the use of INT in glucose assays. Rittersdorf et al, German DE 2,147,466 describes the use of seven 2-(2-benzothiazolyl)-3-phenyl-5-(4-[trimethylammonio]phenyl) tetrazolium salts in the determination of reducing substances such as reducing sugars, ascorbic acid, and ketosteroids.

The variety of 2-thiazolyl tetrazolium salts and/or their corresponding formazans known in the literature are represented by the following. Serebryakova et al, Khim. Geterotsikl. Soedin. 10:1403-1405 (1970) desribe the synthesis and chromatic properties of benzothiazolyl-3-phenyl (methyl)-5-p-nitro(dimethylamino)phenylformazans. The authors state that both an electron-withdrawing nitro group at the para-position of the N-5 phenyl and a benzothiazolyl group at the N-1 position provides a bathochromic shift. Bednyagina et al, Khim. Geterotsikl. Soedin. 2:342-345 (1967) desribe the synthesis of 1-benzothiazolyl- and 1-benzoxazolyl-3-methyl- (or phenyl) 5-arylformazans. The relationship between the depth of color and basicity of the heterocycle is also discussed. Gulemina et al, Khim. Geterotsikl. Soedin. 6:774-777 (1974) describe the effect of an o-nitro group on the structure and properties of benzothiazolylformazans. The ionization constants and IR and UV spectra were determined and related to structure. Lipunova et al, Khim. Geterotsikl. Soedin. 4:493-499 (1974) describe the preparation of 3-(methyl, isopropyl, phenyl)-1-benzothiazolyl-5-nitrophenylformazans and their spectral properties. Gluemina et al, Zh. Prikl. Spektrosk. 22(5):941-943 (1975) describe the observation of both positive and negative UV thermochromism, depending upon solvent and conditions of temperature change, for

1-benzothiazolyl-, 1-thiazolyl-, and 1-(5-bromothiazolyl)-3-methyl-5-(p-nitrophenyl) formazans. Ponyaev et al, Zh. Obshch. Khim. 55(11):2615-2617 (1985) describe kinetic studies of interconversion of the photoinduced forms of 1-benzothiazolyl-3-methyl-5-phenylformazan analogs. It was shown that these forms are more deeply colored than triphenylformazan. Lipunova et al, Khim. Geterotsikl. Soedin. (1971) 831-835 compare the bathochromic effect of a 5-naphthyl or o-tolyl group on the visible spectrum of 1-benzothiazolylformazans.

Various 2-benzothiazolyl tetrazolium salts and related compounds have also been applied to the photographic field, as represented by the descriptions in U.S. Pat. Nos. 3,655,382 and 4,221,864.

## SUMMARY OF THE INVENTION

The present invention provides 2-benzothiazolyl tetrazolium salts which upon reduction yield formazans having new and improved optical properties. The compounds of the present invention are selected from the group consisting of 2-(benzothiazol-2-yl)-3-(4-nitrophenyl)-5-(4-acetamidophenyl)tetrazolium salt;

2-(benzothiazol-2-yl)-3-(4-methoxyphenyl)-5-(4-acetamidophenyl) tetrazolium salt;
2-(6-ethoxybenzothiazol-2-yl)-3-(4-nitrophenyl)-5-(4-acetamidophenyl) tetrazolium salt;
2-(6-ethoxybenzothiazol-2-yl)-3-(4-nitro-1-naphthyl)-5-(4-acetamidophenyl) tetrazolium salt;
2-(6-ethoxybenzothiazol-2-yl)-3-(8-quinolyl)-5-(4-acetamidophenyl) tetrazolium salt;
2-(6-ethoxybenzothiazol-2-yl)-3-(1-naphthyl)-5-(4-acetamidophenyl) tetrazolium salt;
2-(6-ethoxybenzothiazol-2-yl)-3-(1-naphthyl)-5-(4-nitrophenyl) tetrazolium salt;
2-(6-ethoxybenzothiazol-2-yl)-3-(2-methyl-4-carboxyphenyl)-5-(4-acetamidophenyl) tetrazolium salt;
2-(6-ethoxybenzothiazol-2-yl)-3-(4-carboxyphenyl)-5-(4-acetamidophenyl) tetrazolium salt;
2-(6-ethoxybenzothiazol-2-yl)-3-(4-chloro-1-naphthyl)-5-(4-acetamidophenyl) tetrazolium salt;
2-[(5,6,7,8)-tetrahydronaphto(2,3-d)thiazolyl-2-yl]-3-(4-nitronaphthyl)-5-(4-methoxyphenyl) tetrazolium salt;
2-[(5,6,7,8)-tetrahydronaphtho(2,3-d)thiazol-2-yl]-3-(4-nitronaphthyl)-5-(3,4-methylenedioxyphenyl)tetrazolium salt;
2-[(5,6,7,8)-tetrahydronaphtho(2,3-d)thiazol-2-yl]-3-(4-nitronaphthyl)-5-(4-fluorophenyl) tetrazolium salt;
2-[(5,6,7,8)-tetrahydronaphtho(2,3-d)thiazol-2-yl]-3-(4-nitrophenyl)-5-(4-methoxyphenyl) tetrazolium salt;
2-(6-methylbenzotiazol-2-yl)-3-(3,4,5-trimethoxyphenyl)-5-(4-acetamidophenyl) tetrazolium salt;
2-(6-methylbenzothiazol-2-yl)-3-(3,4,5-trimethoxyphenyl)-5-(3,4,5-trimethoxyphenyl) tetrazolium salt;
2-(6-methylbenzothiazol-2-yl)-3-(4-methoxyphenyl)-5-(4-acetamidophenyl) tetrazolium salt;
2-(6-carboxybenzothiazol-2-yl)-3-(3,4,5-trimethoxyphenyl)-5-(3,4-methylenedioxyphenyl) tetrazolium salt;
2-[naphtho(1,2-d)-thiazol-2-yl]-3-(2-methoxy-4-carboxyphenyl-5-[4-(2-(2-hydroxyethoxy)-ethoxy)-ethoxy)-phenyl] tetrazolium salt;
2-[naphtho(1,2-d)-thiazol-2-yl]-3-(4-acetamidophenyl)-5-(methylenedioxyphenyl) tetrazolium salt;
2-[naphtho(1,2-d)-thiazol-2-yl]-3-(8-quinolyl)-5-(3,4,5-trimethoxyphenyl) tetrazolium salt;
2-[naphtho(1,2-d)-thiazol-2-yl]-3-(2-methoxyphenyl)-5-(3,4-methylenedioxyphenyl) tetrazolium salt;
2-[naphtho(1,2-d)-thiazol-2-yl]-3-(4-nitronaphthyl)-5-(3,4-methylenedioxyphenyl) tetrazolium salt;
2-[naphtho(1,2-d)-thiazol-2-yl]-3-(4-nitronaphthyl)-5-phenyl tetrazolium salt;
2-[naphtho(1,2-d)-thiazol-2-yl]-3-(4-nitrophenyl)-5-[4-(2',3'-dihydroxypropylthio)phenyl] tetrazolium salt;
2-[naphtho(1,2-d)-thiazol-2-yl]-3-(4-carboxyphenyl)-5-(4-acetamidophenyl) tetrazolium salt;
2-[naphtho(1,2-d)-thiazol-2-yl]-3-(4-carboxyphenyl)-5-[4-(2-(2-(2-hydroxyethoxy)ethoxy)ethoxy)phenyl]tetrazolium salt;
2-[naphtho(1,2-d)-thiazol-2-yl]-3-(2-methyl-4-carboxphenyl)-5-(3,4-methylenedioxyphenyl) tetrazolium salt;
2-[naphtho(1,2-d)-thiazol-2-yl]-3-(2-methyl-4-nitrophenyl)-5-(3,4-methylene-dioxyphenyl) tetrazolium salt; and
2-[naphtho(1,2-d)-thiazol-2-yl]-3-(4-cyanophenyl)-5-(3-methoxyphenyl) tetrazolium salt.

The present compounds are characterized by a reflectance spectrum exhibiting an extended plateau above 600 m, preferably above about 650 nm. Such reflectance plateau confers improved accuracy to reflectance-read strip analytical assays, particularly where the optical measurement system has a variable central wavelength.

The compounds of the present invention are useful for the detection of a reducing substance, preferably NADH.

Thus the invention further relates to a method for the detection of a reducing substance which comprises introducing one or more of the tetrazolium salts of the present invention into a medium suspected of containing the reducing substance and determining the presence of the reducing substance by a color change in the tetrazolium salt.

In addition the invention provides a test strip for the detection of a reducing substance comprising one or more of the compounds of the present invention.

BRIEF DESCRIPTION OF THE DRAWINGS

Figs. 1 - 4 show the reflectance spectra of the formazans produced upon reduction of the prior art tetrazolium salts INT, MTT, NBT, and 2-(benzothiazol-2-yl)-3-(1-naphthyl)-5-phenyl tetrazolium salt (USSR) at various concentrations of glucose.

Figs. 5 - 10 show the corresponding spectra for the formazans from particular indicator compounds of the present invention (see list at the beginning of the Table in the Examples section.

## Counteranion

The selection of the counteranion will be based primarily on considerations of stability and solubility of the particular tetrazolium salt of interest. In general, one can select from such counteranions as the inorganic anions chloride, bromide, iodide, nitrate, fluoroborate,perchlorate, and sulfate, as well as organic anions such as acetate, oxalate, tartrate, and aryl sulfonates (benzene sulfonate, tosylate).

## Synthetic Methods

Tetrazolium salts are prepared by methods well known in the literature (Hooper, W.D., Rev. Pure and Appl. Chem., 1969, 19, 221; Putter, R., in Methoden der Organischen Chemie, Houben-Weyl-Muller ed., Thieme Verlag: Stuttgart, 1965, Bd. 10/3, p. 633; Nineham, A. W. Chem. Rev., 1955, pp. 355-483). In general, the tetrazolium salts of the present invention are prepared by first reacting a 2-hydrazinothiazole with an aldehyde and then treating the resulting hydrazone with a diazotized aniline. The resulting formazan is then oxidized to the tetrazolium salt by well known methods. Consequently, the synthesis involves three principal starting materials, the aldehyde, the aniline, and the 2-hydrazinothiazole.

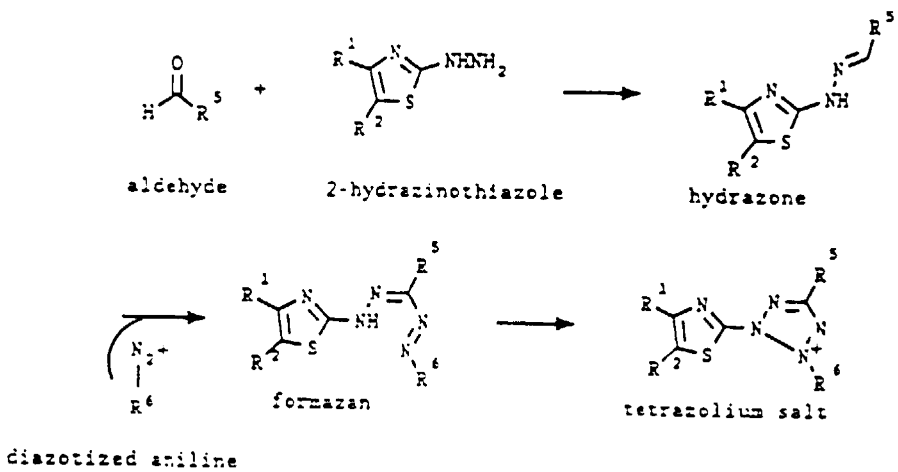
aldehyde
2-hydrazinothiazole
hydrazone
formazan
tetrazolium salt
diazotized aniline

## Preparation of 2-hydrazinothiazoles

The appropriately substituted 2-hydrazino-benzothiazoles are prepared from 1-halo-2-nitro arenes (1) by first displacement of the halide with thiocyanate to produce the nitrothiocyanate (2). This is then reduced with reducing agents such as tin, and the resulting aminothiocyanate (3) cyclized to the 2-aminobenzothiazole (4).

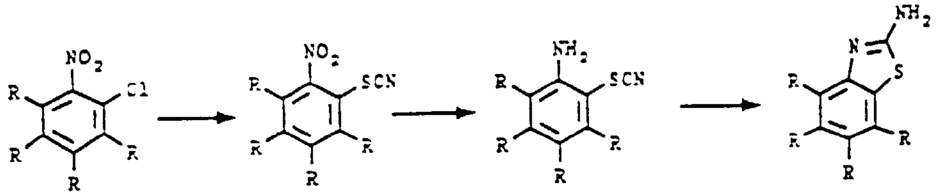

1 2 3 4

The same aminothiocyanate (3) can also be synthesized by treating the amine with thiocyanogen (Metzger, J.V., in Comprehensive Heterocyclic Chemistry, A.R. Katritzky, C.W. Rees ed.; Peragamon: New York, 1984; Vol. 6, Part 4b, p. 324).

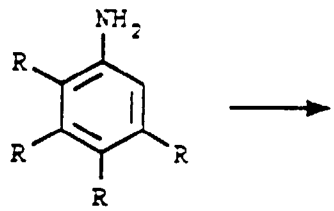

3

The 2-hydrazinothiazole (5) is then prepared by reacting hydrazine with the 2-aminothiazole (4) in ethylene glycol at 140° C (Liu, K-C., Shih, B-J., Arch. Pharm., 1985, 18, 283).

4

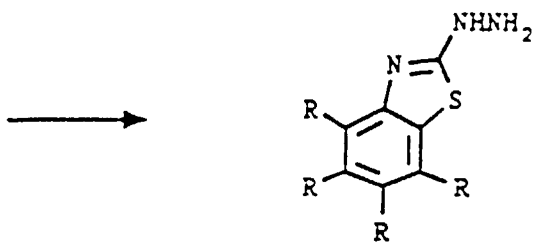

5

Other methods of preparing 2-aminobenzothiazoles (4) are reacting the aromatic amine (6) with mineral acid and thiocyanate to produce the thiourea (8) (Org. Synth., Coll. Vol. IV, 180) or reacting the aromatic amine with N-benzoylthiocyanate to form the N-benzoylthiourea (7) and then hydrolyzing the benzoyl group to produce the thiourea (Org. Synth., Coll. Vol., III, 635). Other methods of thiourea preparation can also be found in these references. These thioureas are then oxidized with reagents such as bromine to produce 2-aminobenzothiazoles [Bhargave, P.N., Baliga B.T., J. Ind. Chem., 1958, 5, 807).

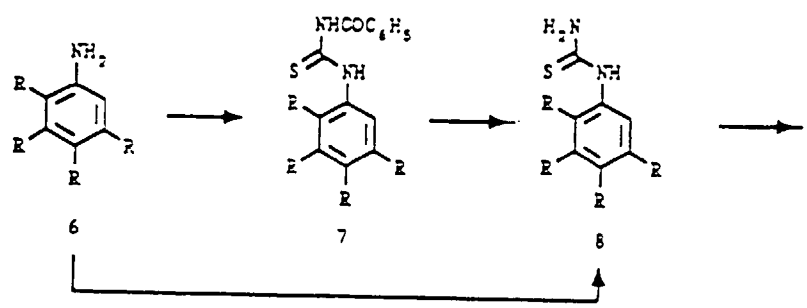

6 7 8

4

Preparation of aldehydes

The aldehydes are obtained from commercial sources or can be prepared by methods familiar to one of ordinary skill in the art.

For instance, aldehydes may be prepared by benzylic oxidation of an arylmethane (March, J., Advances Organic Chemistry Third Edition; John Wiley and Sons: New York, 1985; p. 1079), reduction of an aryl acid chloride (Ibid. p. 396) or aryl acid derivative, [Larock, R.C., Comprehensive Organic Transformations; VCH: New York, 1989; pp. 604-605).

Aryl halides may also be used to synthesize aldehydes. In this method, a transmetallation reaction produces an arylmetallic species which can be treated with a variety of reagents, such as dimethylformamide, to produce the aldehyde (ibid, p. 681-683).

The aforementioned aryl aldehydes, acids, methanes, and halides, may be derivatized with a variety of functional groups prior to their transformation into tetrazolium salts. This may be accomplished by aromatic nucleophilic substitution (March, J., Advanced Organic Chemistry Third Edition; John Wiley and Sons: New York, 1985; pp. 576-607), aromatic electrophilic substitution (ibid., pp. 447-511) or heteroatom-directed metallation reactions (Gschwend, H.W., Rodriguez, H.R., in Organic Reactions, John Wiley and Sons: New York, 1979; Vol. 26, 1).

In cases where the aldehyde piece of the tetrazolium salt contains a phenol or amine, the groups must be protected so that there is not a reaction between these and the diazotized aniline or oxidizing reagent used to prepare the tetrazolium salt.

This can be accomplished by protecting a hydroxyaryl aldehyde as an acetate, performing the reaction sequence to make the formazan, and then hydrolyzing the acetate at pH 10. Acidification to pH 5 and then filtration produces the desired formazan.

Where the resulting phenol formazan reacts with oxidizing agent in the tetrazolium salt preparation, the phenol may be protected by an acid labile group such as dihydropyran (Greene, T.W., Protective Groups in Organic Synthesis, John Wiley and sons: New York; 1981, pp. 87-113) and which is removed by stirring the tetrazolium salt in acidic conditions.

Similarly amines on the aldehyde piece must be protected to prevent their reaction. This is best accomplished by using an acid labile carbamate (ibid, pp. 218-247) which is later removed by stirring the tetrazolium salt under acidic conditions.

Preparation of aryl amines

Aryl amines may be prepared by reduction of the corresponding nitro or azide compound (Larock, P.C., Comprehensive Organic Transformations; VCH: New York, 1989; pp. 412-415 or 409-410), reaction between an arylmetallic compound and an electrophilic nitrogen reagent, (ibid., pp. 399-400), or rearrangement of acyl azides or oxidized amides (ibid., pp. 431-432).

As in the aldehyde case, electrophilic and nucleophilic aromatic substitution can be used to introduce different functional groups into the aryl amine or synthetic precursor.

Use of the Compounds

The principal use of the tetrazolium salt compounds of the present invention are as chromogenic indicators for detecting reducing substances. In particular, the present compounds are advantageous in the detection of NADH. As such, since NADH is produced in enzyme-catalyzed detection reactions specific for various biochemical substances, the present compounds are particularly useful in medical diagnostic tests. However, in general other reducing substances can also be detected, such as hydrogen sulfide gas, diborane, arsenic hydride, or phosphorus hydride.

The present compounds have been particularly found to exhibit an extended plateau in their reflectance spectra above about 600 nm. The most preferred indicator compounds of the present invention have a plateau above about 650 nm (i.e., the flatest about 50 nm wide portion begins between 640 and 660 nm). Such a reflectance plateau confers improved accuracy to analytical tests based on the measurement of reflectance from a reagent strip.

Reagent strips are known in the art as analytical devices comprising a solid carrier matrix incorporated with a test composition that produces a color change in response to contact with a liquid test sample containing the analyte of interest. Such test composition, in the case of the present invention, comprises (a) a reagent or reagents which react with the analyte to produce a reducing substance, and (b) a 2-thiazolyl tetrazolium salt as described herein which is reducable by such reducing substance to produce a chromogenic formazan dye product. The color response of such reagent strips can be observed visually to give semi-quantitative values, however, quantitative results are obtained by measuring the reflectance of the carrier matrix at a predetermined wavelength. Such measurements involve irradiating the reacted carrier matrix with a light source and sensing the reflectance of the carrier matrix by measuring reflected

light with a detector element.

The finding of tetrazolium salt indicators having a reflectance plateau is used to particular advantage where reflectance from a reagent strip is read using an instrument which is subject to variability in the central wavelength of its optical system (the combination of light source, detector element, spectral control elements, e.g., filters , and other components). Variability in the central wavelength of the optical system can be caused by a variety of factors, for example, variability in the central wavelength of the principal spectral control element such as the illuminating light source or filters. For instance, where light emitting diodes (LEDs) are used as the light source, the wavelength of emitted light will typically vary ± 4 nm within an instrument, and up to ± 8 nm between LEDs in different instruments, due to manufacturing variability. Moreover, LEDs are suceptible to variable central wavelength due to temperature effects as well. Where broad band light sources are used with filters to provide spectral control of the central wavelength, variability within an instrument is typically under 1 nm, however, between instrument variability can be as high as ± 6 nm. Thus, the present invention is applicable in those situations where the central wavelength of the light reaching the detector element in the instrument is susceptible to variations in the range of about ± 5 nm.

In the making of a reagent strip for use in the present invention, selection of the carrier matrix, the test reagents which react with analyte to produce the reducing substance, and the method by which such reagents and the tetrazolium indicator are incorporated with the carrier matrix are matters well known in the art of reagent strips. For the sake of reciting just a few examples, typical carrier matrices are porous and absorbent paper, cloth, glass fiber filters, polymeric membranes and films, and the like. Incorporation methods include impregation of a formed carrier matrix with a solution, suspension, or other liquid form of the test composition, in one or more steps, followed by drying of the matrix; formation of a matrix in the presence of one or more of the components of the test composition, e.g., by casting or layering solutions of film or membrane forming formulations.

The present invention will now be illustrated, but is not intended to be limited by, the following examples.

## EXAMPLES

### A. Compound Synthesis

#### Hydrazone Preparation

A slurry of 25 mmole of the appropriate aldehyde and 25 mmole of the appropriate hydrazine in 125 mL of absolute ethanol, is refluxed for 3 hours. Water is removed with 3A molecular seives in a Soxhlet extractor. The mixture is cooled to room temperature and then filtered to yield the hydrazone.

#### Formazan Preparation

The diazonium salt is first prepared by cooling a slurry or solution of 8.5 mmol of the amine in 60 mL of 3 N HCl to 5°C. Sodium nitrite (0.70 g, 10.15 mmol) in 5 mL of water is then added dropwise. After stirring for 30 minutes, the mixture added dropwise to a cold (-25°C) mixture of 8.5 mmol of the hydrazone in 120 mL of 1:1 (v/v) DMF-pyridine. The reaction is not allowed to warm beyond -15°C during the addition. The mixture is allowed to warm to room temperature while stirring for two hours. Filtration produces the formazan as a black solid. Impurities can be removed by repeated washing with methanol or refluxing the solid in methanol and filtering while hot.

#### Tetrazolium Salt Preparation

A slurry of 1.5 mmol of the formazan is stirred with 20 mL of acetic acid and 4 mL of isoamyl nitrite for a period of 16-48 hours. The mixture is then filtered to yield the tetrazolium salt. In cases where the salt does not precipitate, dilution with ether caused precipitation.

### B. Preparation of Reagent Strips

Each indicator was impregnated into a reagent strip and tested with a solution containing a known quantity of glucose, cholesterol, or NADH. The reagent strip consists of a polystyrene handle onto which a single reagent pad is attached. The reagent pad was 0,5 cm x 0,5 cm (0.2 x 0.2") square and contains reagents allowing for a color change which was instrumentally read when an aliqout of sample containing the appropriate analyte was applied. The dry-phase reagent pad is a solid support composed of a cellulosic fibers or a nylon membrane as examples. The reagent pad was impregnated first with a solution of the tetrazolium salt of interest (0.8M/L) and detergent (0.3 %) in a solvent such as methanol. The second solution impregnate into the reagent pad contains the following components:

Glucose Strips

| | |
|---|---|
| Glucose Dehydrogenase (GDH) | 0.8U/L |
| Diaphorase (DPH) | 0.8U/L |
| NAD | 0.03 Mol/L |
| PIPES Buffer | 0.15 Mol/L |
| Detergent | 0.5% |

Cholesterol Strips

| | |
|---|---|
| Cholesterol Dehydrogenase (CDH) | 0.3U/L |
| Cholesterol Ester Hydrolase (CEH) | 0.6U/L |
| Diaphorase | 0.3U/L |
| NAD | 0.04Mol/L |
| Pipes Buffer | 0.2Mol/L |
| Detergent | 1%v/v |

About 0.01 ml of several test solutions (serum, plasma, aqueous) containing at least five different analyte concentrations between 0 and 33 nM was applied to the center of the dried reagent pad. After a lag time of about 60 seconds, the reflectance spectra of each indicator was measured at 5 nm increments over the wavelength range of 400 to 1100 nanometers.

C. Utility data

Following is a table of spectral and other analytical data pertaining to various synthesized tetrazolium salts of the present invention and to various synthesized comparison examples. The compounds are represented by the general formula (A) :

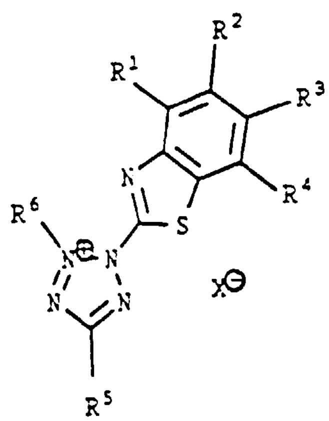

The compounds are organized, in order, by the form of their benzothiazolyl residue, then by their $R^6$ substituent and finally by their $R^5$ substituent. For example, the first compound presented is A.1.a) and is of the Formula A wherein all of $R^1$ - $R^4$ are hydrogen (forming an unsubstituted benzothiazolyl ring), $R^6$ is 4-nitrophenyl, and $R^5$ is 4-acetamidophenyl; the second compound, A.1.b), has the same benzothiazolyl residue and the same $R^6$ substituent as compound A.1.a) but with $R^5$ being phenyl; and so forth.

The reflectance spectrum of tetrazolium salts is understood to be dependent upon the environment in which they are observed or measured. For purposes of comparison between individual tetrazolium salts, the data below include a measurement of the relative flatness of the flatest portion of reflectance spectrum at wavelengths greater than 600 nm, which spectrum is generated using a glucose or cholesterol reagent strip prepared as described in Part B above. The relative flatness of the spectrum is expressed in the data n K/S units normalized for the level of analyte detected

as defined below.

K/S is defined by the equation

$$\frac{(1\text{-}R)^2}{2R}$$

wherein R is instrumentally read reflectance units. Percent change in K/S is the change, expressed as a percentage, over a 50 nm range divided by the average of the high and low K/S values over such range.

The plateau property of the present compounds shall be understood, for the purposes of this invention, as a percent change in reflectance spectrum (expressed in terms of K/S as defined in the paragraph above) of less than about 17% over a 30-50 nm wavelength span beginning at a wavelength above about 600 nm. The more preferable compounds exhibit a plateau having a percent change in K/S of less than an about 10% over a 50 nm wavelength span. Most preferred are those tetrazolium salt indicators exhibiting a percent change in K/S of about 5% or less over a 50 nm wavelength span. Compounds having a more sloped reflectance spectrum are nonetheless preferred where the flatest portion is at wavelengths above 650 nm, preferably above 675 nm.

With reference to the drawings, Figs. 1-4 show the reflectance spectra of the formazans produced upon reduction of the prior art tetrazolium salts INT, MTT, and NBT at various concentrations of glucose. For purposes of comparison, Figs. 5-10 show the corresponding spectra for selected compounds of the present invention. The presence of a plateau in the spectra of the formazans from the present compounds, and its absence from that of the formazans from the prior art compounds is readily apparent.

The four above-mentioned prior art compounds exhibit percent changes in K/S over the wavelength range 650-700 nm as follows:

| | |
|---|---|
| INT | 71% |
| MTT | 178% |
| NBT | 73% |
| USSR | 28% |

The lower the percent K/S value for the formazan, the more tolerant is the tetrazolium salt to variations in the central wavelength of the optical system used to measure reflectance, and hence to measure analyte concentration.

The following non-standard abbreviations are used in the text below:

"UV" - The wavelength in nanometers of maximum reflectance peak in the UV reflectance spectrum of the formazan. The extinction coefficient and solvent used during measurement are given in parentheses.

"nm" - The position of the flatest portion of the reflectance spectrum of the formazan over a 50 nm wavelength span (expressed as the beginning and ending wavelengths in nanometers).

"K/S" The percent change in K/S units over the above mentioned flatest 50 nm portion of the reflectance spectrum. The concentration of analyte used to generate the reflectance spectrum is given in parentheses. Mmol refers to the concentration in mmol/liter.

TABLE

Following is a list of the compounds whose reflectance spectra are shown in Figs. 5-10 of the drawings. Their shorthand reference number and location in the table that follows are given in brackets ahead of the compound names.

Fig. 5 [A-2-b] 2-(Benzothiazol-2-yl)-3-(4-methoxyphenyl)-5 acetamido-phenyl tetrazolium salt
Fig. 6 [B-6-a] 2-(6-Ethoxybenzothiazol-2-yl)-3-(1-naphthyl)-5-(4-acetamidophenyl) tetrazolium salt
Fig. 7 [G-1-a] 2-(Naphtho[1,2-d]thiazol-2-yl)-3-(2-methoxy-4-carboxyphenyl)-5-(3,4-methylenedioxyphenyl) tetrazolium salt
Fig. 8[G-17-a] 2-(Naphtho[1,2-d]thiazol-2-yl)-3-(4-carboxyphenyl)-5-(4-acetamidophenyl) tetrazolium salt
Fig. 9 [G-23-a] 2-(Naphtho[1,2-d]thiazol-2-yl)-3-(4-methylthiophenyl)-5-(4-acetamidophenyl) tetrazolium salt
Fig. 10 [H-1-a] 2-(8-Methoxy[1,2-d]naphthol-2-yl)-3-(3,4,5-trimethoxyphenyl)-5-(4-methoxyphenyl) tetrazolium salt

A. Benzothiazol-2-yl

1. $R^6$ = 4-nitrophenyl

a) $R^5$ = 4-acetamidophenyl

| | |
|---|---|
| UV: | 478 nm (7.8x$10^3$, dioxane); |
| nm: | 620 - 670 nm; K/S: 9% (11.1 mmol) |
| IR(KBr): | 3441, 1691, 1684, 1614, 1600, 1535, 1486, 1460, 1425, 1385, 1318, 1262, 1183, 979, 853 $cm^{-1}$ |
| NMR (300 NHz, $CDCl_3$): | δ 8:67 (d, 2H, J=8.9Hz, $ArNO_2$ H), 8.33 (d, 2H, J=9.0 Hz), 8.36 (d, 2H, J=9.3 Hz, ArH), 8.37 - 8.41 (m, 1H, ArH), 7.99 (d, 2H, J=8.7 Hz, ArH) 7.96 - 7.99 (m, 1H, benzothiazole H), 7.67 - 7.78 (m, 3H, benzothiazole H). |
| Mass Spectrum FAB m/e: | 120 (81.1), 162 (100) |

b) $R^5$ = phenyl

UV: 540 nm (4.79 x $10^3$, dioxane);

c) $R^5$ = phenyl

UV: 576 nm (3.89 x $10^3$, dioxane)
nm: 650 - 700 nm; K/S: 7.5% (11.1 mmol)

d) $R^5$ = 4-nitrostyryl

UV: 512 nm (11.2 x $10^3$, dioxane)

e) $R^5$ = 4-methoxystyryl

UV: 514 nm (7.17 x $10^3$, dioxane)

f) $R^5$ = 4-nitrophenyl

UV: 542 nm (2.1 x $10^3$, dioxane)

g) $R^5$ = 4-phenylphenyl

UV: 558 nm (14.1 x $10^3$, dioxane)

h) $R^5$ = 3,4-methylenedioxyphenyl

UV: 584 nm (4.8 x $10^3$, dioxane)
nm: 570 - 620 nm; K/S: 16% (9.1 mmol)

i) $R^5$ = 4-phenoxyphenyl

UV: 564 nm (10.3 x $10^3$, dioxane)

j) $R^5$ = 2-thienyl

UV: 566 nm (6.3 x $10^3$, dioxane)

k) $R^5$ = 5-nitro-2-thienyl

UV: 514 nm (15.2 x $10^3$, dioxane)

l) $R^5$ = 3,4-dimethoxyphenyl

UV: 518 nm (2.3 x $10^3$, dioxane)
nm: 600 - 650 nm; K/S: 14% (11.1 mmol)

m) $R^5$ = 4-pyridyl

UV: 516 nm (8.6 x $10^3$, dioxane)

n) $R^5$ = 5-bromo-2-thienyl

UV: 518 nm (6.1 x $10^3$, dioxane)

o) $R^5$ = 3-thienyl

UV: 512 nm (7.5 x $10^3$, dioxane)

p) $R^5$ = 2-pyridyl

UV: 478 nm (9.6 x $10^3$, dioxane)

q) $R^5$ = 4-fluorophenyl

UV: 546 nm (2.96 x $10^3$, dioxane)

r) $R^5$ = 4-hydroxyphenyl

UV: 582 nm (1.0 x $10^3$, dioxane)

s) $R^5$ = 3,4,5-trimethoxyphenyl

UV: 578 mm (1.0 x $10^3$, dioxane)
nm: 560 - 610 nm; K/S: 10% (11.1 mmol)

t) $R^5$ = 4-(methylthio)phenyl

UV: 576 nm (1.5 x $10^3$, dioxane)

u) $R^5$ = 1,4-benzodioxyl

UV: 582 nm (4.8 x $10^3$, dioxane)

2. $R^6$ = 4-methoxyphenyl Ex.: A.2. b) Comp. Ex.: A.2. a)

a) $R^5$ = phenyl

UV: 468 (14.5 x $10^3$, dioxane) 476 (water)
nm: 625 - 675 nm; K/S: 15% (22 mmol)

b) $R^5$ = 4-acetamidophenyl

nm: 665 - 715 nm; K/S: 10% (33 mmol)

3. $R^6$ = 4-methoxy-1-naphthyl Comp. Ex.: A.3 a)

a) $R^5$ = phenyl

UV: 504 (7.8 x $10^3$, dioxane) 495 (water)

4. $R^6$ = 4-bromophenyl Comp. Ex.: A.4. a)

a) $R^5$ = 4-acetamidophenyl

UV: 480 ($7.7 \times 10^3$, dioxane)

B. 6-ethoxybenzothiazol-2-yl Ex.: B.1. a) Comp. Exs.: B.1. b) to d)

1. $R^6$ = 4-nitrophenyl

a) $R^5$ = 4-acetamidophenyl

| | |
|---|---|
| UV: | 598 nm ($3.2 \times 10^3$, dioxane) |
| nm: | 625 - 675 nm; K/S: 9% (14 mmol) |
| IR(KBr): | 3447, 1601, 1535, 1459, 1255, 1183, 853 $cm^{-1}$ |
| NMR ($CDCl_3$): | δ 8.42 - 8.70 (5H), 8.16 - 8.32 (2H), 8.03 - 8.12 (1H), 7.69 - 7.84 (2H), 7.16 - 7.27 (1H benzothiazol), 4.18 (q, 2H, J = 6.9 Hz), 2.56 (q, 2H, J = 7.4 Hz), 1.46 (t, 3H, J = 6.9 Hz), 1.17 (t, 3H, J - 7.4 Hz). |

b) $R^5$ = 4-hydroxyphenyl

UV: 601 nm ($13 \times 10^3$, dioxane)

c) $R^5$ = 4-(2,3-dihydroxypropylthio)phenyl

UV: 604 nm ($8.4 \times 10^3$, dioxane)

d) $R^5$ = 4-(trimethylammonium)phenyl

UV: 604 nm ($5.0 \times 10^3$, dioxane)
nm: 540 - 590 nm; K/S: 15% (11.1 mmol)

2. $R^6$ = phenyl Comp. Exs.: B.2. a) to c)

a) $R^5$ = 4-(dihydroxypropylthio)phenyl

UV: 530 nm ($1.0 \times 10^3$, dioxane)

b) $R^5$ = 4-(trimethylammonium)phenyl

UV: 530 nm ($8.3 \times 10^3$, dioxane)

c) $R^6$ = 4-acetamidophenyl

UV: 545 (water)
nm: 620 - 670 nm; K/S: 18% (11.1 mmol)

3. $R^6$ = 4-nitro-1-naphthyl Ex.: B.3. a) Comp. Ex.: B.3. b)

a) $R^5$ = 4-acetamidophenyl

UV: 612 mm ($3.4 \times 10^3$, dioxane)
nm: 675 - 725 nm; K/S: 5% (14.1 mmol)

b) $R^5$= (4-trimethylammonium)phenyl

UV: 634 mm ($4.6 \times 10^3$, dioxane)
nm: 640 - 690 nm; K/S: 15% (11.1 mmol)

4. $R^6$ = 8-quinolyl Ex.: B.4. a)

a) $R^5$ = 4-acetamidophenyl

| | |
|---|---|
| UV: | 628 mm ($12.1 \times 10^3$, 420) |
| nm: | 650 - 700 nm; K/S: 8% (8.1 mmol) |
| IR(KBr): | 3436, 1684, 1600, 1531, 1458, 1423, 1385, 1318, 1258, 959, 939, 834 $cm^{-1}$ |
| NMR (DMSO-$d_6$): | $\delta$ 10.8 (s, 1H, NH), 8.67 - 8.83 (m, 3H), 8.34 (d, 24, J = 8.8 Hz, pH), 8.11 (t, 1H, J = 7.8 Hz), 8.04 (d, 2H, J = 8.8 Hz, pH), 7.87 (d, 1H, J = 2.5 Hz), 7.74 (dd, 1H, J = 8.4 Hz, 4.3 Hz), 7.46 (1H, J = 9.1 Hz), 7.12 (dd, 1H, J = 9.0 Hz, 2.6 Hz), 4,10 (q, 2H, J = 7.0 Hz, -$CH_2$-Me), 2.26 (s, 34, $CH_3$-CO), 1.35 (t, 3H, J = 7.0 Hz, Me). |
| Mass Spectrum (FAB) m/e: | 508 ($M^+$-1, 18.7), 353 (24.1), 128 (100). |

5. $R^6$ = 3,4-methylenedioxyphenyl Comp. Ex.: B.5 a)

a) $R^5$ = 4-methylenedioxyphenyl

| | |
|---|---|
| UV: | 504 nm ($\varepsilon$ $5.9 \times 10^3$, dioxane) |
| nm: | 670 - 720 nm; K/S: 14% (11.1 mmol) |
| IR(KBr): | 3465, 1602, 1540, 1501, 1462, 1384, 1254, 1179, 1117, 1037, 932, 813 $cm^{-1}$ |
| NMR (300 MHz, DMSO-$d_6$): | $\delta$ 8.01 (d, 1H, J = 9.08), 7.93 (dd, 1H, J = 8.14 Hz, 1.79 Hz), 7.92 (d, 1H, J = 2.46 Hz), 7.53 - 7.61 (m, 2H), 7.26 - 7.36 (m, 3H), 6.34 (s, 2H, -$OCH_2O$-), 6.26 (s, 2H, -$OCH_2O$-), 4.17 (q, 2H), J = 6.99 Hz, -$CH_2CH_3$), 1.40 (t, 3H, J = 6.94 Hz, -$CH_2CH_3$), |
| Mass spectrum (FAB) m/e: | 93.1 (100), 185.2 (27.8), 340 (16.9), 488 (3.0, $M^+$-1) 489 (1.4, $M^+$), 490 (7.6, $M^+$+1). |

6. $R^6$ = 1-naphthyl Ex.: B.6, a), b)

a) $R^5$ = 4-acetamidophenyl

| | |
|---|---|
| UV: | 532 ($13 \times 10^3$, dioxane) 632 (water) |
| nm: | 660 - 710; K/S: 3% (11.1 mmol) |
| $^1$HNMR (300 MHz, 80:20 $CDCl_3$/DMSO-$d_6$): | 10.35 (s, 1H), 8.40 (d, J = 8, 1H), 8.40 (d, J = 8, 1H), 8.28 (m, 2H), 8.17 (d, J = 8, 1H), 8.03 (d, J = 8.5, 2H), 7.95 (d, J = 8, 1H), 7.8-7.6 (m, 3H), 7.58 (d, J = 7.5, 1H) 7.45 (d, J = 9, 1H), 7.08 (dd, J = 2.59, 9, 1H), 4.13 (q, J = 7, 2H), 2.2 (s, 3H), 1.43 (t, J = 7, 3H). |

b) $R^5$ = 4-nitrophenyl

UV: 600 ($8.4 \times 10^3$, dioxane) 620 (water)
nm: 645 - 695 nm; K/S: 7% (9.4 mmol)

7. $R^6$ = 2-methyl-4-carboxyphenyl Ex.: B.7 a)

a) $R^5$ = 4-acetamidophenyl

UV: 600-630 ($11 \times 10^3$, dioxane)
nm: 615 - 665; K/S: 5% (11.1 mmol)

8. $R^6$ = 4-carboxyphenyl Ex.: B.8. a)

a) $R^5$ = 4-acetamidophenyl

UV: 592 ($9.5 \times 10^3$, dioxane) 610 (water)
nm: 615 - 665 nm; K/S: 2% (8.1 mmol)

9. $R^6$ = 4-methoxyphenyl Comp. Ex.: B.9. a)

a) $R^5$ = phenyl

UV: 488 (14.5 x $10^3$, dioxane)

10. $R^6$ = 4-chloro-1-naphthyl Ex.: B.10 a)

a) $R^5$ = 4-acetamidophenyl

UV: 600 (water)
nm: 610 - 660; K/S: 3% (8.3 mmol)

11. $R^6$ = 4-cyano-1-naphthyl Comp. Ex.: B.11. a)

a) $R^5$ = 4-acetamidophenyl

UV: 632 (9.1 x $10^3$, dioxane) 606 (water)
nm: 625 - 675; K/S: 11% (8.3 mmol)

C. (5,6,7,8)-Tetrahydronaphtho[2,3-d]thiazol-2-yl

1. $R^6$ = 4-nitronaphthyl Exs.: C.1. b) to d) Comp. Ex.: C.1. a)

a) $R^5$ = 4-acetamidophenyl

| | |
|---|---|
| UV: | 624 nm (5.1 x $10^3$, dioxane) |
| nm: | 635 - 685 nm; K/S: 14% (14 mmol) |
| IR(KBr): | 1635, 1540, 1500, 1470, 1440, 1390, 1260, 1120, 1040, 920 $cm^{-1}$ |
| NMR (300 KHz, $CDCl_3$): | $\delta$ 8.99 - 9.12 (m, 1H, naphthalene), 8.60 (d, 1H, J = 8.6 Hz), 8.25 (d, 1H, J = 4.9 Hz, naphthalene) 7.72 - 7.94 (m, 6H, naphthalene and benzothiazole), 7.55 (d, 2H, J = 8.0 Hz, PhNHAC), 7.12 - 7.23 (m, 2H, naphthalene), 7.01 (d, 2H, J = 8.4 Hz, PhNHAC), 2.70 - 2.90 (broad S, 4H, $-CH_2-$), 1.80 - 2.95 (m, 7H, $CH_3CO$- and $-CH_2-$). |
| Mass Spectrum (E2) m/e: | 121 (22.4), 149 (100, 165 (34.7), 165 (34.7), 164 (20.7), 205 (26.2), 349 ($K^+$ $-1-C_{10}H_2NO_2$, 12.1) |
| Mass Spectrum (FAB) m/e: | 350 ($M^+$ - $C_{10}H_2NO_2$, 100). |

b) $R^5$ = 4-methoxyphenyl

UV: 590 nm (9.0 x $10^3$, dioxane)
nm: 640 - 690 nm; K/S: 7.1% (14 mmol)

c) $R^5$ = 3,4-methylenedioxyphenyl

UV: 626 nm (1.2 x $10^3$, dioxane)
nm: 660 - 710 nm; K/S: 5% (9.1 mmol)

d) $R^5$ = 4-fluorophenyl

UV: 612 mm (3.4 x $10^3$, dioxane)
nm: 625 - 675 nm; K/S: 8.5% (14 mmol)

2. $R^6$ = 4-nitrophenyl Ex.: C.2. b) Comp. Exs.: C.2. a), c), d)

a) $R^5$ = 4-acetamidophenyl

UV: 596 nm (2.0 x $10^3$, dioxane)
nm: 640 - 690 nm; K/S: 16% (14 mmol)

b) $R^5$ = 4-methoxyphenyl

UV: 590 nm (9.0 x $10^3$, dioxane)
nm: 645 - 695 nm; K/S: 8% (14 mmol)

c) $R^5$ = 3,4-methylenedioxyphenyl

UV: 598 mm (4.4 x $10^3$, dioxane)
nm: 640 - 690 nm; K/S: 20% (14 mmol)

d) $R^5$ = 4-hydroxyphenyl

UV: 598 nm (6.0 x $10^3$, dioxane)

3. $R^6$ = 1-naphthyl Comp. Ex.: C.3. a)

a) 4-(2-(2-(2-ethoxy)ethoxy)ethoxy)phenyl

UV: 436 nm (12 x $10^3$, $H_2O$)

D. 6-methylbenzothiazol-2-yl EX.: D.1. b), c) Comp. Ex.: D-1, a)

1. $R^6$ = 3,4,5-trimethoxyphenyl

a) $R^5$ = 3,4-methylenedioxy

| | |
|---|---|
| UV: | 492 nm (4.7 x $10^3$, dioxane) |
| nm: | 655 - 705 nm; K/S: 20% (11.1 mmol) |
| IR(KBr): | 1604, 1540, 1496, 1464, 1432, 1370, 1317, 1254, 1123, 1037, 993, 822, 770, 735 $cm^{-1}$ |
| NMR (300 MHz, DMSO-d6): | δ 8.17 (5, 1H, PhH), 8.01 (d, 1H, J = 8.48 Hz). 7.95 (dd, 1H, J = 8.13 Hz, 1.76 Hz), 7.85 (d, 1H, J = 1.72 Hz), 7.57 (dd, 1H, J = 8.19 Hz), 1.53 Hz), 7.49 (s, 2H), 7.32 (d, 1H, J = 8.16 Hz), 6.30 (s, 24, $-OCH_2-O$), 3.85 (s, 3H, -OMe), 3.79 (s, 6H, OMe), 2.54 (s, 3H, $-CH_3$) |
| Mass Spectrum (FAB) m/e: | 93.2 (100, 185 (62.8), 310 (25.9), 325 (4.1), 504 (10.9, $M^+$-1) |

b) $R^5$ = 4-acetamidophenyl

UV: 492 mm (3.6 x $10^3$, dioxane)
nm: 650 - 700 nm; K/S: 10% (11.1 mmol)

c) $R^5$ = 3,4,5-trimethoxyphenyl

UV: 492 nm (4.9 x $10^3$, dioxane)
nm: 655 - 705 nm; K/S: 8% (11.1 mmol)

2. $R^6$ = 4-methoxyphenyl Ex.: D.2. a)

a) $R^5$ = 4-acetamidophenyl

| | |
|---|---|
| UV: | 488 nm (3.9 x $10^3$, dioxane) |
| nm: | 650 - 700 nm; K/S: 8% (17.7 mmol) |
| IR(KBr): | 1690, 1599, 1531, 1501, 1459, 1422, 1370, 1317, 1265, 1181, 1164, 1021, 977, 837, 747, 695 $cm^{-1}$ |
| NMR (300 MHz, DNSO-$d_6$): | δ 10.45, (s, 1H, -NHAc), 8.30 (d, 2H, J = 8.75 Hz, Ph-H), 8.17 (s, 1H, PhH), 7.90 - 8.11 (m, 5H), 7.26 - 7.40 (m, 1H), 3.84 (s, 3H, -OMe), 2.50 (s, 3H, -Me), 2.14 (s, 3H, $CH_3$) |
| Mass Spectrum (FAB) m/e: | 93, 100, 149 (8.1), 163 (19.9), 185 (91.1), 323 (21.6) 457 (7,1, $M^+$-1) |

E. 6-carboxybenzothiazol-2-yl

1. $R^6$ = 3,4,5-trimethoxyphenyl Ex.: E.1. a)

a) $R^5$ = 3,4-methylenedioxyphenyl

| | |
|---|---|
| UV: | 408 nm (4.9 x $10^3$, dioxane) |
| nm; | 665 - 735 nm; K/S: 10% (11.1 mmol) |
| IR(KBr): | 3405, 3200-2500 (broad), 1700, 1602, 1500, 1451, 1259, 1122, 1034, 990, 815, 771 $cm^{-1}$ |
| NMR (300 MHz, DMSO-$d_6$): | δ 8.57 (s, 1H, PhH), s, 7.95 dd, 1H, J = 8.09, 1.68 Hz), 7.85 (d, 1H, J = 1.70 Hz), 7.17 - 7.43 (m, 4H), 7.02 (d, 1H, J = 8.0 Hz), 6.11 (s, 2H), 3.90 (s, 3H, OMe), 3.74 (s, 6H, OMe) |
| Mass Spectrum (FAB) m/e: | 93.1 (57.1), 167.2 (100), 185.2 (50.8), 340.1 (48.1, $M^+$ - $C_8H_5NSO_2$) |

2. $R^6$ = 4-nitrophenyl Comp. Ex.: E.2. a)

a) $R^5$ = 3,4-methylenedioxyphenyl

UV: 568 nm (0.6 x $10^3$, dioxane)

F. 5,6,7-trimethoxybenzothiazol-2-yl

1. $R^6$ = 4-carboxyphenyl Comp. Ex.: F.1. a)

a) $R^5$ = 4-methoxyphenyl

| | |
|---|---|
| UV: | 594 (dioxane) 516 (water) |
| nm: | 630 - 680; K/S: 19% (8.3 mmol) |
| $^1H$ NMR (DMSO$d_6$): | 8.33 (m, 3H), 8.20 (d, J = 7, 2H), 7.35 (m, 3H), 4.10 (s, 3H), 3.95 (s, 3H), 3.85 (s, 6H) |

G. naphtho[1,2-d]thiazol-2-yl

1. $R^6$ = 2-methoxy-4-carboxyphenyl Ex. G.1.b) Comp. Ex.: G.1. a)

a) $R^5$ = 3,4-methylenedioxyphenyl

| | |
|---|---|
| UV: | 568 nm (4.5 x $10^3$, 420) |
| nm: | 650 - 700 nm; K/S: 12% (11.1 mmol) |
| IR(KBr): | 1713, 1605, 1499, 1466, 1433, 1315, 1259, 1209, 1037, 741 $cm^{-1}$ |
| NMR (300 MHz, DMSO-$d_6$): | δ 8.42 (d, 1H, J = 9.0 Hz, -Ph), 8.17 - 8.17 (m, 3H), 8.00 - 7.96 (m, 2H), 7.87 - 7-66 (m, 5H), 7.33 (d, 1H, J = 8.1 Hz), 6.30 (s, 2H, $OCH_2O$), 3.82 (s, 3H, -OMe) |
| Mass Spectrum (FAB) m/e: | 93 (100, 185 (47), 346 (18), 524 (23, $M^+$-1) |

b ) $R^5$ = 4-(2-(2-(hydroxyethoxy)-ethoxy)-ethoxy)-phenyl

UV: 566 nm (3.2 x $10^3$. $H_2O$)
nm: 650 - 700 nm; K/S: 6% (11.1 mmol)

2. $R^6$ = 4-acetamidophenyl Ex.: G.2. c) Comp. Ex.: G.2. a), b)

a) $R^5$ = 4-cyanophenyl

| | |
|---|---|
| UV: | 524 nm (9 x $10^3$, $H_2O$) |
| nm: | 650 - 700 nm; K/S: 16% (8.1 mmol) |
| IR(KBr): | 3437, 3047, 1694, 1594, 1537, 1503, 1462, 1414, 1384, 1265, 1172, 985, 848 $cm^{-1}$ |

NMR (300 MHz, DMSO-$d_6$): δ 10.85 (s, 1H, NHAc), 8.59 (d, 24, J - 8.4 Hz), 8.41 (d, 1H, J = 9.0 Hz), 8.30 - 8.64 (m, 94), 7.79 - 7.76 (m, 2H), 2.18 (s, 3H, $-NHCOCH_3$)
Mass Spectrum (FAB) m/e: 91 (100), 109 (28, 181 (56), 217 (45), 232 (13), 327 (8), 488 (10, $M^+$-1), 487 (4, $M^+$)

b) $R^5$ = 4-carbomethoxyphenyl

UV: 558 nm (12 x $10^3$, 420)
nm: 660 - 710 nm; K/S 25% (8.3 mmol)

c) $R^5$ = methylenedioxyphenyl

nm: 685 - 735; K/S: 7% (8.1 mmol)

3. $R^6$ = 3,4,5-trimethoxyphenyl Comp. Ex.: G.3. a) to c)

a) $R^5$ = 4-(3-trimethylammoniopropoxy)phenyl

UV: 520 (11 x $10^3$, water)
nm: 670 - 720; K/S: 13% (8.3 mmol)

b) $R^5$ = 3,4-methylenedioxyphenyl

UV: 565 nm (10.3 x $10^3$, $H_2O$)
nm; 675 - 725 nm; K/S: 20% (8.3 mmol)

c) $R^5$ = 4-(2-(2-(2-hydroxy)ethoxy)ethoxy) phenyl

UV: 592 nm (11.4 x $10^3$, $H_2O$)

4. $R^6$ = 3,4-methylenedioxy Comp. Ex.: G.4. a)

a) $R^5$ = 3,4-methylenedioxy

UV: 544 nm (10.7 x $10^3$, $H_2O$)
nm: 685 - 735 nm; K/S: 26% (8.3 mmol)

5. $R^6$ = 4-methoxyphenyl Comp. Ex.: G.5. a)

a) $R^5$ = 3,4-methylenedioxyphenyl

UV: 546 nm (7.9 x $10^3$, $H_2O$)
nm: 675 - 725 nm; K/S: 14% (8.3 mmol)

6. $R^6$ = 1-anthryl Comp, Ex.: G.6. a)

a) $R^5$ = 3,4-methylenedioxyphenyl

UV: 644 nm (4.1 x $10^3$, $H_2O$)
nm: 750 - 800 nm; K/S: 32% (8.3 mmol)

7. $R^6$ = 8-quinolyl Ex.: G.7. b) Comp. Ex.: G.7. a)

a) $R^5$ = 3,4-methylenedioxyphenyl

UV: 640 nm (3.9 x $10^3$, $H_2O$)
nm: 650 - 700 nm; K/S: 5% (8.3 mmol)

b) $R^5$ = 3,4,5-trimethoxyphenyl

UV: 628 nm (12.1 x $10^3$, $H_2O$)
nm: 650 - 700 nm; K/S: 4% (8.3 mmol)

8. $R^6$ = 2,5-dimethoxy-4-nitrophenyl Comp. Ex.: G.8. a)

a) $R^5$ = 3,4-methylenedioxy

UV: 654 nm (7.0 x $10^3$, $H_2O$)
nm: 655 - 705 nm; K/S: 17% (8.3 mmol)

9. $R^6$ = 2-methoxy-4-carbomethoxyphenyl Comp. Ex.: G.9. a)

a) $R^5$ = 3,4-methylenedioxyphenyl

UV: 630 nm (6.3 x $10^3$, $H_2O$)
nm: 650 - 700 nm; K/S: 12% (8.3 mmol)

10. $R^6$ = 2-methoxy-5-acetamidophenyl Comp. Ex.: G.10. a)

a) R5 = 3,4-methylenedioxyphenyl

UV: 584 nm (7.0 x $10^3$, $H_2O$)
nm: 660 - 710 nm; K/S: 11% (8.3 mmol)

11. $R^6$ = 2-methoxyphenyl Ex.: G.11. a)

a) $R^5$ = 3,4-methylenedioxyphenyl

UV: 618 nm (10.4 x $10^3$, $H_2O$)
nm: 650 - 700 nm; K/S: 10% (8.3 mmol)

12. $R^6$ = 2,4-dimethoxyphenyl Comp. Ex.: G.12. a)

a) $R^5$ = 3,4-methylenedioxyphenyl

nm: 675 - 725 nm; K/S: 33% (8.3 mmol)

13. $R^6$ = 2-methoxy-4-nitrophenyl Comp. Ex.: G.13. a)

a) $R^5$ = 3,4-methylenedioxyphenyl

UV: 650 nm (14 x $10^3$, $H_2O$)
nm: 650 - 700 nm; K/S: 14% (8.3 mmol)

14. $R^6$ = 2,5-dimethoxyphenyl Comp. Ex.: G.14. a)

a) $R^5$ = 3,4-methylenedioxy

nm: 665 - 715 nm; K/S: 36% (8.3 mmol)

15. $R^6$ = 4-nitro-naphthyl Ex.: G.15. b), c) Comp. Ex.: G.15. a), d), e)

a) $R^5$ = 4-fluorophenyl

UV: 622 (15.6 x $10^3$, dioxane) 632 (water)
nm: 625 - 725 nm; K/S: 12% (13.8 mmol)

b) $R^5$ = 3,4-methylenedioxyphenyl

UV: (13.1 x $10^3$, dioxane)
nm: 605 - 655 nm; K/S: 4% (15 mmol)

c) $R^5$ = phenyl

UV: 618 (12.6 x $10^3$, dioxane)
nm: 625 - 675; K/S = 8% (14 mmol)

d) $R^6$ = 4-(2-(2-(2-hydroxyethoxy)ethoxy) phenyl

UV: 628 nm (6.7 x $10^3$, $H_2O$)

e) $R^6$ = 4-(1'-(hydroxymethyl)-2',3'-dihydroxypropylthio)phenyl

UV: 624 nm (5.18 x $10^3$, dioxane)
nm: 635 - 685 nm; K/S: 15% (11.1 mmol)

16. $R^6$ = 4-nitrophenyl Ex.: G.16. a) Comp. Ex.: G.16.b)

a) $R^5$ = 4-(2',3'-dihydroxypropylthio)phenyl

UV: 604 nm (4.9 x $10^3$, dioxane)
nm: 635 - 685 nm; K/S: 4% (11.1 mmol)

b) $R^5$ = 4-(trimethylammoniun)phenyl

UV: 572 nm (14.7 x $10^3$, dioxane)

17. $R^6$ = 4-carboxyphenyl Ex.: G.17. a), d) Comp. Ex.: G.17. b), c), e)

a) $R^5$ = 4-acetamidophenyl

NMR(DMSO-$d_6$): δ 10.46 (s, 1H), 8.5 - 8.22 (m, 1H), 8.17 (d, J = 8, 1H), 8.02 (d, J = 8.8, 2H), 7.85 (dd, J = 7.6, 0.9, 1H), 7.78 - 7.65 (m, 3H), 2.08 (s, 3H)
UV: 598 (dioxane) 614 (10.5 x $10^3$, water)
nm: 650 - 700 nm; K/S: 7.5% (13.8 mmol)

b) $R^5$ = 4-(3-trimethylammoniopropoxy)phenyl

UV: 632 (14.7 x $10^3$, $H_2O$)
nm: 640 - 690; K/S: 11% (8.6 mmol)

c) $R^5$ = 4-(2',3'-dihydroxypropylthio)phenyl

UV: 654 nm (1.0 x $10^3$, dioxane)
nm: 645 - 695 nm; K/S: 11% (11.1 mmol)

d) $R^5$ = 4-(2-(2-(2-hydroxyethoxy)ethoxy) ethoxy)phenyl

nm: 625 - 675 nm; K/S: 6.5% (8.3 mmol)

e) $R^5$ = carbmethoxyphenyl

nm: 605 - 655; K/S: 11.5% (8.3 mmol)

18. $R^6$ = 2-methyl-4-carboxyphenyl Ex.: G.18. a)

a) $R^5$ = 3,4-methylenedioxyphenyl

UV: 620 - 640 (dioxane) 614 (8.4 x $10^3$, water)
nm: 625 - 675 nm; K/S: 3% (14 mmol)

19. $R^6$ = 2-methyl-4-nitrophenyl Ex.: G.19. a)

a) $R^5$ = 3,4-methylenedioxyphenyl

UV: 644 (11.3 x $10^3$, dioxane) 625 (9.2 x $10^3$, water)
nm: 665 - 715 nm; K/S: 6% (14 mmol)

20. $R^6$ = phenyl Comp, Ex.: G.20. a)

a) $R^5$ = 4-acetamidophenyl

UV: 516 (7.1 x $10^3$, dioxane) 526 (7.2 x $10^3$, water)

21. $R^6$ = 4-methoxyphenyl Comp. Ex.: G.21. a)

a) $R^5$ = 4-acetamidophenyl

UV: 502 (9.5 x $10^3$, dioxane) 500 (water)

22. $R^6$ = 4-phenylazophenyl Comp. Ex.: G.22. a)

a) $R^5$ = 4-methoxyphenyl

UV: 614 (dioxane) 618 (water
nm: 625 - 675 nm; K/S: 11% (8.3 mmol)

23. $R^6$ = 4-methylthiophenyl Comp. Ex.: G.23. a)

a) $R^5$ = 4-acetamidophenyl

UV: 524 (dioxane) 526 (water)
nm: 675 - 725 nm; K/S: 18% (8.3 mmol)

24. $R^6$ = 4-(3,4-dihydroxybutylamido)phenyl Comp. Ex.: G.24. a)

a) $R^5$ = 4-methoxyphenyl

UV: 588 (9 x $10^3$, dioxane) 578 (12 x $10^3$, water)

25. $R^6$ = 4-carbmethoxyphenyl Comp. Ex.: G.25. a)

a) $R^5$ = 4-methoxyphenyl

UV: 598 (9.4 x $10^3$, dioxane) 581 (9.1 x $10^3$, water)
nm: 630 - 680 nm; K/S: 20% (8.3 mmol)

26. $R^6$ = 4-cyanophenyl Ex.: G.26. a)

a) $R^5$ = 3-methoxyphenyl

UV: 602 (12.3 x $10^3$, dioxane) 608 (water)
nm; 625 - 675 nm; K/S: 8% (8.3 mmol)

27. $R^6$ = 4-benzamido-5-methyl-2-methoxy-phenyl Comp. Ex.: G.27. a)

e) $R^5$ = 3,4-methylenedioxyphenyl

nm: 690 - 740; K/S: 15% (10.3 mmol)

28. $R^6$ = 4-benzamido-2,5-dimethoxyphenyl Comp. Ex.: G.28. a)

a) $R^5$ = 3,4-methylenedioxyphenyl

nm: 715 - 765; K/S: 17% (10.3 mmol)

H. 8-methoxynaphtho[1,2-d]thiazol-2-yl Comp. Ex.: H.1. a)

1. $R^6$ = 3,4,5-trimethoxyphenyl

a) $R^5$ = 4-methoxyphenyl

| | |
|---|---|
| UV: | 466 (9.2 x $10^3$, dioxane) 530 (9 x $10^3$, water) |
| nm: | 670 - 720 nm; K/S: 12% (8.3 mmol) |
| NMR (300 MHz, DMSO-$d_6$): | $\delta$ 8.33 (m, 3H), 7.97 (m, 1H), 7.90 (s, 1H), 7.75 (m, 2H), 7.55 (s, 2H), 7.35 (d, J = 9, 2H), 4.10 (s, 3H), 3.93 (s, 3H), 3.88 (s, 3H), 3.78 (s 6H) |

## Claims

**Claims for the following Contracting States : AT, BE, CH, DE, DK, FR, GB, IT, LI, LU, NL, SE**

**1.** 2-Benzothiazol tetrazolium salts which are selected from the group consisting of 2-(benzothiazol-2-yl)-3-(4-nitrophenyl)-5-(4-acetamidophenyl)tetrazolium salt;

2-(benzothiazol-2-yl)-3-(4-methoxyphenyl)-5-(4-acetamidophenyl) tetrazolium salt;
2-(6-ethoxybenzothiazol-2-yl)-3-(4-nitrophenyl)-5-(4-acetamidophenyl) tetrazolium salt;
2-(6-ethoxybenzothiazol-2-yl)-3-(4-nitro-1-naphthyl)-5-(4-acetamidophenyl) tetrazolium salt;
2-(6-ethoxybenzothiazol-2-yl)-3-(8-quinolyl)-5-(4-acetamidophenyl) tetrazolium salt;
2-(6-ethoxybenzothiazol-2-yl)-3-(1-naphthyl)-5-(4-acetamidophenyl) tetrazolium salt;
2-(6-ethoxybenzothiazol-2-yl)-3-(1-naphthyl)-5-(4-nitrophenyl) tetrazolium salt;
2-(6-ethoxybenzothiazol-yl)-3-(2-methyl-4-carboxyphenyl)-5-(4-acetamidophenyl) tetrazolium salt;
2-(6-ethoxybenzothiazol-2-yl)-3-(4-carboxyphenyl)-5-(4-acetamidophenyl) tetrazolium salt;
2-(6-ethoxybenzothiazol-2-yl)-3-(4-chloro-1-naphthyl)-5-(4-acetamidophenyl) tetrazolium salt;
2-[(5,6,7,8)-tetrahydronaphto(2,3-d)thiazolyl-2-yl]-3-(4-nitronaphthyl)-5-(4-methoxyphenyl)tetrazolium salt;
2-[(5,6,7,8)-tetrahydronaphtho(2,3-d)thiazol-2-yl]-3-(4-nitronaphthyl)-5-(3,4-methylenedioxyphenyl)tetrazolium salt;
2-[(5,6,7,8)-tetrahydronaphtho(2,3-d)thiazol-2-yl]-3-(4-nitronaphthyl)-5-(4-fluorophenyl) tetrazolium salt;
2-[(5,6,7,8)-tetrahydronaphtho(2,3-d)thiazol-2-yl]-3-(4-nitrophenyl)-5-(4-methoxyphenyl) tetrazolium salt;
2-(6-methylbenzothiazol-2-yl)-3-(3,4,5-trimethoxyphenyl)-5-(4-acetamidophenyl) tetrazolium salt;
2-(6-methylbenzothiazol-2-yl)-3-(3,4,5-trimethoxyphenyl)-5-(3,4,5-trimethoxyphenyl) tetrazolium salt;
2-(6-methylbenzothiazol-2-yl)-3-(4-methoxyphenyl)-5-(4-acetamidophenyl) tetrazolium salt;
2-(6-carboxybenzothiazol-2-yl)-3-(3,4,5-trimethoxyphenyl)-5-(3,4-methylenedioxyphenyl) tetrazolium salt;
2-[naphtho(1,2-d)-thiazol-2-yl]-3-(2-methoxy-4-carboxyphenyl-5-[4-(2-(2-hydroxyethoxy)-ethoxy)-ethoxyphenyl] tetrazolium salt;
2-[naphtho(1,2-d)-thiazol-2-yl]-3-(4-acetamidophenyl)-5-(methylenedioxyphenyl) tetrazolium salt;
2-[naphto(1,2-d)-thiazol-2-yl]-3-(8-quinolyl)-5-(3,4,5-trimethoxyphenyl) tetrazolium salt;
2-[naphtho(1,2-d)-thiazol-2-yl]-3-(2-methoxyphenyl)-5-(3,4-methylenedioxyphenyl) tetrazolium salt;
2-[naphtho(1,2-d)-thiazol-2-yl]-3-(4-nitronaphthyl)-5-(3,4-methylenedioxyphenyl) tetrazolium salt;
2-[naphtho(1,2-d)-thiazol-2-yl]-3-(4-nitronaphthyl)-5-phenyl tetrazolium salt;
2-[naphtho(1,2-d)-thiazol-2-yl]-3-(4-nitrophenyl)-5-[4-(2',3'-dihydroxypropylthio)phenyl] tetrazolium salt;

2-[naphto(1,2-d)-thiazol-2-yl]-3-(4-carboxyphenyl)-5-(4-acetamidophenyl) tetrazolium salt;
2-[naphtho(1,2-d)-thiazol-2-yl]-3-(4-carboxyphenyl)-5-[4-(2-(2-(2-hydroxyethoxy)ethoxy)ethoxy)phenyl] tetrazolium salt;
2-[naphtho(1,2-d)-thiazol-2-yl]-3-(2-methyl-4-carboxphenyl)-5-(3,4-methylenedioxyphenyl)tetrazolium salt;
2-[naphtho(1,2-d)-thiazol-2-yl]-3-(2-methyl-4-nitrophenyl)-5-(3,4-methylene-dioxyphenyl)tetrazolium salt; and
2-[naphtho(1,2-d)-thiazol-2-yl]-3-(4-cyanophenyl)-5-(3-methoxyphenyl) tetrazolium salt.

**2.** A method for the detection of a reducing substance which comprises introducing one or more of the tetrazolium salts of claim 1 into a medium suspected of containing the reducing substance and determining the presence of the reducing substance by a color change in the tetrazolium salt.

**3.** Use of the compounds of claim 1 for the detection of a reducing substance, preferably NADH.

**4.** A reagent for the detection of a reducing substance comprising one or more of the compounds according to claim 1.

**5.** A test strip for the detection of a reducing substance comprising one or more of the compounds according to claim 1.

**Claims for the following Contracting States : ES, GR**

**1.** A method for the detection of a reducing substance which comprises introducing one or more 2-benzothiazol tetrazolium salts which are selected from the group consisting of 2-(benzothiazol-2-yl)-3-(4-nitrophenyl)-5-(4-acetamidophenyl) tetrazolium salt;

2-(benzothiazol-2-yl)-3-(4-methoxyphenyl)-5-(4-acetamidophenyl) tetrazolium salt;
2-(6-ethoxybenzothiazol-2-yl)-3-(4-nitrophenyl)-5-(4-acetamidophenyl) tetrazolium salt;
2-(6-ethoxybenzothiazol-2-yl)-3-(4-nitro-1-naphthyl)-5-(4-acetamidophenyl) tetrazolium salt;
2-(6-ethoxybenzothiazol-2-yl)-3-(8-quinolyl)-5-(4-acetamidophenyl) tetrazolium salt;
2-(6-ethoxybenzothiazol-2-yl)-3-(1-naphthyl)-5-(4-acetamidophenyl) tetrazolium salt;
2-(6-ethoxybenzothiazol-2-yl)-3-(1-naphthyl)-5-(4-nitrophenyl) tetrazolium salt;
2-(6-ethoxybenzothiazol-2-yl)-3-(2-methyl-4-carboxyphenyl)-5-(4-acetamidophenyl)tetrazolium salt;
2-(6-ethoxybenzothiazol-2-yl)-3-(4-carboxyphenyl)-5-(4-acetamidophenyl) tetrazolium salt;
2-(6-ethoxybenzothiazol-2-yl)-3-(4-chloro-1-naph-thyl)-5-(4-acetamidophenyl) tetrazolium salt;
2-[(5,6,7,8)-tetrahydronaphto(2,3-d)thiazolyl-2-yl]-3-(4-nitronaphthyl)-5-(4-methoxyphenyl)tetrazolium salt;
2-[(5,6,7,8)-tetrahydronaphtho(2,3-d)thiazol-2-yl]-3-(4-nitronaphthyl)-5-(3,4-methylenedioxyphenyl) tetrazolium salt;
2-[(5,6,7,8)-tetrahydronaphtho(2,3-d)thiazol-2-yl]-3-(4-nitronaphthyl)-5-(4-fluorophenyl)tetrazolium salt;
2-[(5,6,7,8)-tetrahydronaphtho(2,3-d)thiazol-2-yl]-3-(4-nitrophenyl)-5-(4-methoxyphenyl)tetrazolium salt;
2-(6-methylbenzothiazol-2-yl)-3-(3,4,5-trimethoxyphenyl)-5-(4-acetamidophenyl) tetrazolium salt;
2-(6-methylbenzothiazol-2-yl)-3-(3,4,5-trimethoxyphenyl)-5-(3,4,5-trimethoxyphenyl) tetrazolium salt;
2-(6-methylbenzothiazol-2-yl)-3-(4-methoxyphenyl)-5-(4-acetamidophenyl) tetrazolium salt;
2-(6-carboxybenzothiazol-2-yl)-3-(3,4,5-trimethoxyphenyl)-5-(3,4-methylenedioxyphenyl)tetrazolium salt;
2-[naphtho(1,2-d)-thiazol-2-yl]-3-(2-methoxy-4-carboxyphenyl-5-[4-(2-(2-hydroxyethoxy)-ethoxy)-ethoxy)-phenyl] tetrazolium salt;
2-[naphtho(1,2-d)-thiazol-2-yl]-3-(4-acetamidophenyl)-5-(methylenedioxyphenyl) tetrazolium salt;
2-[naphto(1,2-d)-thiazol-2-yl]-3-(8-quinolyl)-5-(3,4,5-trimethoxyphenyl) tetrazolium salt;
2-[naphtho(1,2-d)-thiazol-2-yl]-3-(2-methoxyphenyl)-5-(3,4-methylenedioxyphenyl) tetrazolium salt;
2-[naphtho(1,2-d)-thiazol-2-yl]-3-(4-nitronaphthyl)-5-(3,4-methylenedioxyphenyl) tetrazolium salt;
2-[naphtho(1,2-d)-thiazol-2-yl]-3-(4-nitronaphthyl)-5-phenyl tetrazolium salt;
2-[naphtho(1,2-d)-thiazol-2-yl]-3-(4-nitrophenyl)-5-[4-(2',3'-dihydroxypropylthio)phenyl]tetrazolium salt;
2-[naphto(1,2-d)-thiazol-2-yl]-3-(4-carboxyphenyl)-5-(4-acetamidophenyl) tetrazolium salt;
2-[naphtho(1,2-d)-thiazol-2-yl]-3-(4-carboxyphenyl)-5-[4-(2-(2-(2-hydroxyethoxy)ethoxy)ethoxy)phenyl] tetrazolium salt;
2-[naphtho(1,2-d)-thiazol-2-yl]-3-(2-methyl-4-carboxphenyl)-5-(3,4-methylenedioxyphenyl) tetrazolium salt;
2-[naphtho(1,2-d)-thiazol-2-yl]-3-(2-methyl-4-nitrophenyl)-5-(3,4-methylene-dioxyphenyl)tetrazolium salt; and
2-[naphtho(1,2-d)-thiazol-2-yl]-3-(4-cyanophenyl)-5-(3-methoxyphenyl)tetrazolium salt into a medium suspected of containing the reducing substance and determining the presence of the reducing substance by a color change in the tetrazolium salt.

**2.** Use of the compounds specified in claim 1 for the detection of a reducing substance, preferably NADH.

**3.** A reagent for the detection of a reducing substance comprising one or more of the compounds according to claim 1.

**4.** A test strip for the detection of a reducing substance comprising one or more of the compounds according to claim 1.

**Patentansprüche**

**Patentansprüche für folgende Vertragsstaaten : AT, BE, CH, DE, DK, FR, GB, IT, LI, LU, NL, SE**

**1.** 2-Benzthiazoltetrazoliumsalze, die aus der Gruppe ausgewählt sind, bestehend aus:

2-(Benzthiazol-2-yl)-3-(4-nitrophenyl)-5-(4-acetamidophenyl)tetrazoliumsalz,
2-(Benzthiazol-2-yl)-3-(4-methoxyphenyl)-5-(4-acetamidophenyl)tetrazoliumsalz,
2-(6-Ethoxybenzthiazol-2-yl)-3-(4-nitrophenyl)-5-(4-acetamidophenyl)tetrazoliumsalz,
2-(6-Ethoxybenzthiazol-2-yl)-3-(4-nitro-1-naphthyl)-5-(4-acetamidophenyl)tetrazoliumsalz,
2-(6-Ethoxybenzthiazol-2-yl)-3-(8-chinolyl)-5-(4-acetamidophenyl)tetrazoliumsalz,
2-(6-Ethoxybenzthiazol-2-yl)-3-(1-naphthyl)-5-(4-acetamidophenyl)tetrazoliumsalz,
2-(6-Ethoxybenzthiazol-2-yl)-3-(1-naphthyl)-5-(4-nitrophenyl)tetrazoliumsalz,
2-(6-Ethoxybenzthiazol-2-yl)-3-(2-methyl-4-carboxyphenyl)-5-(4-acetamidophenyl)tetrazoliumsalz,
2-(6-Ethoxybenzthiazol-2-yl)-3-(4-carboxyphenyl)-5-(4-acetamidophenyl)tetrazoliumsalz,
2-(6-Ethoxybenzthiazol-2-yl)-3-(4-chlor-1-naphthyl)-5-(4-acetamidophenyl)tetrazoliumsalz,
2-[(5,6,7,8)-Tetrahydronaphtho(2,3-d)thiazol-2-yl]-3-(4-nitronaphthyl)-5-(4-methoxyphenyl)tetrazoliumsalz,
2-[(5,6,7,8)-Tetrahydronaphtho(2,3-d)thiazol-2-yl]-3-(4-nitronaphthyl)-5-(3,4-methylendioxyphenyl)tetrazoliumsalz,
2-[(5,6,7,8)-Tetrahydronaphtho(2,3-d)thiazol-2-yl]-3-(4-nitronaphthyl)-5-(4-fluorphenyl)tetrazoliumsalz,
2-[(5,6,7,8)-Tetrahydronaphtho(2,3-d)thiazol-2-yl]-3-(4-nitrophenyl)-5-(4-methoxyphenyl)tetrazoliumsalz,
2-(6-Methylbenzthiazol-2-yl)-3-(3,4,5-trimethoxyphenyl)-5-(4-acetamidophenyl)tetrazoliumsalz,
2-(6-Methylbenzthiazol-2-yl)-3-(3,4,5-trimethoxyphenyl)-5-(3,4,5-trimethoxyphenyl)tetrazoliumsalz,
2-(6-Methylbenzthiazol-2-yl)-3-(4-methoxyphenyl)-5-(4-acetamidophenyl)tetrazoliumsalz,
2-(6-Carboxybenzthiazol-2-yl)-3-(3,4,5-trimethoxyphenyl)-5-(3,4-methylendioxyphenyl)tetrazoliumsalz,
2-[Naphtho(1,2-d)thiazol-2-yl]-3-(2-methoxy-4-carboxyphenyl-5-[4-(2-(2-hydroxyethoxy)ethoxy)ethoxy)phenyl] tetrazoliumsalz,
2-[Naphtho(1,2-d)thiazol-2-yl]-3-(4-acetamidophenyl)-5-(methylendioxyphenyl)tetrazoliumsalz,
2-[Naphtho(1,2-d)thiazol-2-yl]-3-(8-chinolyl)-5-(3,4,5-trimethoxyphenyl)tetrazoliumsalz,
2-[Naphtho(1,2-d)thiazol-2-yl]-3-(2-methoxyphenyl)-5-(3,4-methylendioxyphenyl)tetrazoliumsalz,
2-[Naphtho(1,2-d)thiazol-2-yl]-3-(4-nitronaphthyl)-5-(3,4-methylendioxyphenyl)tetrazoliumsalz,
2-[Naphtho(1,2-d)thiazol-2-yl]-3-(4-nitronaphthyl)-5-phenyltetrazoliumsalz,
2-[Naphtho(1,2-d)thiazol-2-yl]-3-(4-nitrophenyl)-5-[4-(2',3'-dihydroxypropylthio)phenyl]tetrazoliumsalz,
2-[Naphtho(1,2-d)thiazol-2-yl]-3-(4-carboxyphenyl)-5-(4-acetamidophenyl)tetrazoliumsalz,
2-[Naphtho(l,2-d)thiazol-2-yl]-3-(4-carboxyphenyl)-5-[4-(2-(2-(2-hydroxyethoxy)ethoxy)ethoxy)-phenyl]tetrazoliumsalz,
2-[Naphtho(1,2-d)thiazol-2-yl]-3-(2-methyl-4-carboxphenyl)-5-(3,4-methylendioxyphenyl)tetrazoliumsalz,
2-[Naphtho(1,2-d)thiazol-2-yl]-3-(2-methyl-4-nitrophenyl)-5-(3,4-methylendioxyphenyl)tetrazoliumsalz und aus
2-[Naphtho(1,2-d)thiazol-2-yl]-3-(4-cyanophenyl)-5-(3-methoxyphenyl)tetrazoliumsalz.

**2.** Verfahren zum Nachweis einer reduzierenden Substanz, wobei man eines oder mehrere der Tetrazoliumsalze gemäß Anspruch 1 in ein Medium einbringt, von dem anzunehmen ist, daß es die reduzierende Substanz enthält, und man das Vorhandenseit der reduzierenden Substanz durch eine Farbänderung im Tetrazoliumsalz bestimmt.

**3.** Verwendung der Verbindungen gemäß Anspruch 1 zum Nachweis einer reduzierenden Substanz, vorzugsweise von NADH.

**4.** Reagens zum Nachweis einer reduzierenden Substanz, welches eine oder mehrere der Verbindungen gemäß Anspruch 1 enthält.

5. Teststreifen zum Nachweis einer reduzierenden Substanz, welcher eine oder mehrere der Verbindungen gemäß Anspruch 1 aufweist.

**Patentansprüche für folgende Vertragsstaaten : ES, GR**

1. Verfahren zum Nachweis einer reduzierenden Substanz, wobei man ein oder mehrere 2-Benzthiazoltetrazoliumsalze, die aus der Gruppe ausgewählt sind, bestehend aus:

   2-(Benzthiazol-2-yl)-3-(4-nitrophenyl)-5-(4-acetamidophenyl)tetrazoliumsalz,
   2-(Benzthiazol-2-yl)-3-(4-methoxyphenyl)-5-(4-acetamidophenyl)tetrazoliumsalz,
   2-(6-Ethoxybenzthiazol-2-yl)-3-(4-nitrophenyl)-5-(4-acetamidophenyl)tetrazoliumsalz,
   2-(6-Ethoxybenzthiazol-2-yl)-3-(4-nitro-1-naphthyl)-5-(4-acetamidophenyl)tetrazoliumsalz,
   2-(6-Ethoxybenzthiazol-2-yl)-3-(8-chinolyl)-5-(4-acetamidophenyl)tetrazoliumsalz,
   2-(6-Ethoxybenzthiazol-2-yl)-3-(1-naphthyl)-5-(4-acetamidophenyl)tetrazoliumsalz,
   2-(6-Ethoxybenzthiazol-2-yl)-3-(1-naphthyl)-5-(4-nitrophenyl)tetrazoliumsalz,
   2-(6-Ethoxybenzthiazol-2-yl)-3-(2-methyl-4-carboxyphenyl)-5-(4-acetamidophenyl)tetrazoliumsalz,
   2-(6-Ethoxybenzthiazol-2-yl)-3-(4-carboxyphenyl)-5-(4-acetamidophenyl)tetrazoliumsalz,
   2-(6-Ethoxybenzthiazol-2-yl)-3-(4-chlor-1-naphthyl)-5-(4-acetamidophenyl)tetrazoliumsalz,
   2-[(5,6,7,8)-Tetrahydronaphtho(2,3-d)thiazol-2-yl]-3-(4-nitronaphthyl)-5-(4-methoxyphenyl)tetrazoliumsalz,
   2-[(5,6,7,8)-Tetrahydronaphtho(2,3-d)thiazol-2-yl]-3-(4-nitronaphthyl)-5-(3,4-methylendioxyphenyl)-tetrazoliumsalz,
   2-[(5,6,7,8)-Tetrahydronaphtho(2,3-d)thiazol-2-yl]-3-(4-nitronaphthyl)-5-(4-fluorphenyl)tetrazoliumsalz,
   2-[(5,6,7,8)-Tetrahydronaphtho(2,3-d)thiazol-2-yl]-3-(4-nitrophenyl)-5-(4-methoxyphenyl)tetrazoliumsalz,
   2-(6-Methylbenzthiazol-2-yl)-3-(3,4,5-trimethoxyphenyl)-5-(4-acetamidophenyl)tetrazoliumsalz,
   2-(6-Methylbenzthiazol-2-yl)-3-(3,4,5-trimethoxyphenyl)-5-(3,4,5-trimethoxyphenyl)-tetrazoliumsalz,
   2-(6-Methylbenzthiazol-2-yl)-3-(4-methoxyphenyl)-5-(4-acetamidophenyl)tetrazoliumsalz,
   2-(6-Carboxybenzthiazol-2-yl)-3-(3,4,5-trimethoxyphenyl)-5-(3,4-methylendioxyphenyl)-tetrazoliumsalz,
   2-[Naphtho(1,2-d)thiazol-2-yl]-3-(2-methoxy-4-carboxyphenyl-5-[4-(2-(2-hydroxyethoxy)ethoxy)ethoxy)-phenyl]tetrazoliumsalz,
   2-[Naphtho(1,2-d)thiazol-2-yl]-3-(4-acetamidophenyl)-5-(methylendioxyphenyl)tetrazoliumsalz,
   2-[Naphtho(1,2-d)thiazol-2-yl]-3-(8-chinolyl)-5-(3,4,5-trimethoxyphenyl)tetrazoliumsalz,
   2-[Naphtho(1,2-d)thiazol-2-yl]-3-(2-methoxyphenyl)-5-(3,4-methylendioxyphenyl)tetrazoliumsalz,
   2-[Naphtho(1,2-d)thiazol-2-yl]-3-(4-nitronaphthyl)-5-(3,4-methylendioxyphenyl)tetrazoliumsalz,
   2-[Naphtho(1,2-d)thiazol-2-yl]-3-(4-nitronaphthyl)-5-phenyltetrazoliumsalz,
   2-[Naphtho(1,2-d)thiazol-2-yl]-3-(4-nitrophenyl)-5-[4-(2',3'-dihydroxypropylthio)phenyl]tetrazoliumsalz,
   2-[Naphtho(1,2-d)thiazol-2-yl]-3-(4-carboxyphenyl)-5-(4-acetamidophenyl)tetrazoliumsalz,
   2-[Naphtho(1,2-d)thiazol-2-yl]-3-(4-carboxyphenyl)-5-[4-(2-(2-(2-hydroxyethoxy)ethoxy)ethoxy)phenyl]-tetrazoliumsalz,
   2-[Naphtho(1,2-d)thiazol-2-yl]-3-(2-methyl-4-carboxphenyl)-5-(3,4-methylendioxyphenyl)-tetrazoliumsalz,
   2-[Naphtho(1,2-d)thiazol-2-yl]-3-(2-methyl-4-nitrophenyl)-5-(3,4-methylendioxyphenyl)tetrazoliumsalz und aus
   2-[Naphtho(1,2-d)thiazol-2-yl]-3-(4-cyanophenyl)-5-(3-methoxyphenyl)tetrazoliumsalz

   in ein Medium einbringt, von dem anzunehmen ist, daß es die reduzierende Substanz enthält, und man das Vorhandensein der reduzierenden Substanz durch eine Farbänderung im Tetrazoliumsalz bestimmt.

2. Verwendung der in Anspruch 1 spezifizierten Verbindungen zum Nachweis einer reduzierenden Substanz, vorzugsweise von NADH.

3. Reagens zum Nachweis einer reduzierenden Substanz, welches eine oder mehrere der Verbindungen gemäß Anspruch 1 enthält.

4. Teststreifen zum Nachweis einer reduzierenden Substanz, welcher eine oder mehrere der Verbindungen gemäß Anspruch 1 aufweist.

## Revendications

**Revendications pour les Etats contractants suivants : AT, BE, CH, DE, DK, FR, GB, IT, LI, LU, NL, SE**

1. Sels de 2-benzothiazoltétrazolium qui sont choisis parmi le groupe constitué par:

    le sel de 2-(benzothiazol-2-yl)-3-(4-nitrophényl)-5-(4-acétamidophényl)tétrazolium;
    le sel de 2-(benzothiazol-2-yl)-3-(4-méthoxyphényl)-5-(4-acétamidophényl)tétrazolium;
    le sel de 2-(6-éthoxybenzothiazol-2-yl)-3-(4-nitrophényl)-5-(4-acétamidophényl)tétrazolium;
    le sel de 2-(6-éthoxybenzothiazol-2-yl)-3-(4-nitro-1-naphtyl)-5-(4-acétamidophényl)tétrazolium;
    le sel de 2-(6-éthoxybenzothiazol-2-yl)-3-(8-quinolyl)-5-(4-acétamidophényl)tétrazolium;
    le sel de 2-(6-éthoxybenzothiazol-2-yl)-3-(1-naphtyl)-5-(4-acétamidophényl)tétrazolium;
    le sel de 2-(6-éthoxybenzothiazol-2-yl)-3-(1-naphtyl)-5-(4-nitrophényl)tétrazolium;
    le sel de 2-(6-éthoxybenzothiazolyl)-3-(2-méthyl-4-carboxyphényl)-5-(4-acétamidophényl)tétrazolium;
    le sel de 2-(6-éthoxybenzothiazol-2-yl)-3-(4-carboxyphényl)-5-(4-acétamidophényl)tétrazolium;
    le sel de 2-(6-éthoxybenzothiazol-2-yl)-3-(4-chloro-1-naphtyl)-5-(4-acétamidophényl)tétrazolium;
    le sel de 2-[(5,6,7,8)-tétrahydronaphto(2,3-d)-thiazolyl-2-yl]-3-(4-nitronaphtyl)-5-(4-méthoxyphényl)-tétrazolium;
    le sel de 2-[(5,6,7,8)-tétrahydronaphto(2,3-d)-thiazol-2-yl]-3-(4-nitronaphtyl)-5-(3,4-méthylène-dioxyphényl) tétrazolium;
    le sel de 2-[(5,6,7,8)-tétrahydronaphto(2,3-d)-thiazol-2-yl]-3-(4-nitronaphtyl)-5-(4-fluorophényl)-tétrazolium;
    le sel de 2-[(5,6,7,8)-tétrahydronaphto(2,3-d)-thiazol-2-yl]-3-(4-nitronaphtyl)-5-(4-méthoxyphényl)-tétrazolium;
    le sel de 2-(6-méthylbenzothiazol-2-yl)-3-(3,4,5-triméthoxyphényl)-5-(4-acétamidophényl)tétrazolium;
    le sel de 2-(6-méthylbenzothiazol-2-yl)-3-(3,4,5-triméthoxyphényl)-5-(3,4,5-triméthoxyphényl)-tétrazolium;
    le sel de 2-(6-méthylbenzothiazol-2-yl)-3-(4-méthoxyphényl)-5-(4-acétamidophényl)tétrazolium;
    le sel de 2-(6-carboxybenzothiazol-2-yl)-3-(3,4,5-triméthoxyphényl)-5-(3,4-méthylènedioxyphényl)-tétrazolium;
    le sel de 2-[naphto(1,2-d)-thiazol-2-yl]-3-(2-méthoxy-4-carboxyphényl-5-[4-(2-(2-hydroxyéthoxy)-éthoxy)-éthoxyphényl]tétrazolium;
    le sel de 2-[naphto(1,2-d)-thiazol-2-yl]-3-(4-acétamidophényl)-5-(méthylènedioxyphényl)tétrazolium;
    le sel de 2-[naphto(1,2-d)-thiazol-2-yl]-3-(8-quinolyl)-5-(3,4,5-triméthoxyphényl)tétrazolium;
    le sel de 2-[naphto(1,2-d)-thiazol-2-yl]-3-(2-méthoxyphényl)-5-(3,4-méthylènedioxyphényl)tétrazolium;
    le sel de 2-[naphto(1,2-d)-thiazol-2-yl]-3-(4-nitronaphtyl)-5-(3,4-méthylènedioxyphényl)tétrazolium;
    le sel de 2-[naphto(1,2-d)-thiazol-2-yl]-3-(4-nitronaphtyl)-5-(phényl)tétrazolium;
    le sel de 2-[naphto(1,2-d)-thiazol-2-yl]-3-(4-nitrophényl)-5-[4-(2',3'-dihydroxypropylthio)phényl]-tétrazolium;
    le sel de 2-[naphto(l,2-d)-thiazol-2-yl]-3-(4-carboxyphényl)-5-(4-acétamidophényl)tétrazolium;
    le sel de 2-[naphto(1,2-d)-thiazol-2-yl]-3-(4-carboxyphényl)-5-[4-(2-(2-(2-hydroxyéthoxy)éthoxy)-éthoxy)phényl]tétrazolium;
    le sel de 2-[naphto(1,2-d)-thiazol-2-yl]-3-(2-méthyl-4-carboxyphényl)-5-(3,4-méthylènedioxyphényl)tétrazolium;
    le sel de 2-[naphto(1,2-d)-thiazol-2-yl]-3-(2-méthyl-4-nitrophényl)-5-(3,4-méthylènedioxyphényl)tétrazolium; et
    le sel de 2-[naphto(1,2-d)-thiazol-2-yl]-3-(4-cyanophényl)-5-(3-méthoxyphényl)tétrazolium,

2. Procédé pour la détection d'une substance réductrice, qui comprend le fait d'introduire un ou plusieurs sels de tétrazolium selon la revendication 1 dans un milieu que l'on suspecte de contenir la substance réductrice et de déterminer la présence de la substance réductrice par un changement de couleur dans le sel de tétrazolium.

3. Utilisation des composés selon la revendication 1 pour la détection d'une substance réductrice, de préférence NADH.

4. Réactif pour la détection d'une substance réductrice, comprenant un ou plusieurs des composés selon la revendication 1.

5. Bandelette d'essai pour la détection d'une substance réductrice comprenant un ou plusieurs des composés selon la revendication 1.

**Revendications pour les Etats contractants suivants : ES, GR**

1. Procédé pour la détection d'une substance réductrice, qui comprend le fait d'introduire un ou plusieurs sels de 2-benzothiazoltétrazolium qui sont choisis parmi le groupe constitué par:

   le sel de 2-(benzothiazol-2-yl)-3-(4-nitrophényl)-5-(4-acétamidophényl)tétrazolium;
   le sel de 2-(benzothiazol-2-yl)-3-(4-méthoxyphényl)-5-(4-acétamidophényl)tétrazolium;
   le sel de 2-(6-éthoxybenzothiazol-2-yl)-3-(4-nitrophényl)-5-(4-acétamidophényl)tétrazolium;
   le sel de 2-(6-éthoxybenzothiazol-2-yl)-3-(4-nitro-1-naphtyl)-5-(4-acétamidophényl)tétrazolium;
   le sel de 2-(6-éthoxybenzothiazol-2-yl)-3-(8-quinolyl)-5-(4-acétamidophényl)tétrazolium;
   le sel de 2-(6-éthoxybenzothiazol-2-yl)-3-(1-naphtyl)-5-(4-acétamidophényl)tétrazolium;
   le sel de 2-(6-éthoxybenzothiazol-2-yl)-3-(1-naphtyl)-5-(4-nitrophényl)tétrazolium;
   le sel de 2-(6-éthoxybenzothiazolyl)-3-(2-méthyl-4-carboxyphényl)-5-(4-acétamidophényl)tétrazolium;
   le sel de 2-(6-éthoxybenzothiazol-2-yl)-3-(4-carboxyphényl)-5-(4-acétamidophényl)tétrazolium;
   le sel de 2-(6-éthoxybenzothiazol-2-yl)-3-(4-chloro-1-naphtyl)-5-(4-acétamidophényl)tétrazolium;
   le sel de 2-[(5,6,7,8)-tétrahydronaphto(2,3-d)-thiazolyl-2-yl]-3-(4-nitronaphtyl)-5-(4-méthoxyphényl)-tétrazolium;
   le sel de 2-[(5,6,7,8)-tétrahydronaphto(2,3-d)-thiazol-2-yl]-3-(4-nitronaphtyl)-5-(3,4-méthylènedioxyphényl)tétrazolium;
   le sel de 2-[(5,6,7,8)-tétrahydronaphto(2,3-d)-thiazol-2-yl]-3-(4-nitronaphtyl)-5-(4-fluorophényl)-tétrazolium;
   le sel de 2-[(5,6,7,8)-tétrahydronaphto(2,3-d)-thiazol-2-yl]-3-(4-nitronaphtyl)-5-(4-méthoxyphényl)-tétrazolium;
   le sel de 2-(6-méthylbenzothiazol-2-yl)-3-(3,4,5-triméthoxyphényl)-5-(4-acétamidophényl)tétrazolium;
   le sel de 2-(6-méthylbenzothiazol-2-yl)-3-(3,4,5-triméthoxyphényl)-5-(3,4,5-triméthoxyphényl)-tétrazolium;
   le sel de 2-(6-méthylbenzothiazol-2-yl)-3-(4-méthoxyphényl)-5-(4-acétamidophényl)tétrazolium;
   le sel de 2-(6-carboxybenzothiazol-2-yl)-3-(3,4,5-triméthoxyphényl)-5-(3,4-méthylènedioxyphényl)-tétrazolium;
   le sel de 2-[naphto(1,2-d)-thiazol-2-yl]-3-(2-méthoxy-4-carboxyphényl-5-[4-(2-(2-hydroxyéthoxy)-éthoxy)-éthoxyphényl]tétrazolium;
   le sel de 2-[naphto(1,2-d)-thiazol-2-yl]-3-(4-acétamidophényl)-5-(méthylènedioxyphényl)tétrazolium;
   le sel de 2-[naphto(1,2-d)-thiazol-2-yl]-3-(8-quinolyl)-5-(3,4,5-triméthoxyphényl)tétrazolium;
   le sel de 2-[naphto(1,2-d)-thiazol-2-yl]-3-(2-méthoxyphényl)-5-(3,4-méthylènedioxyphényl)tétrazolium;
   le sel de 2-[naphto(1,2-d)-thiazol-2-yl]-3-(4-nitronaphtyl)-5-(3,4-méthylènedioxyphényl)tétrazolium;
   le sel de 2-[naphto(1,2-d)-thiazol-2-yl]-3-(4-nitronaphtyl)-5-(phényl)tétrazolium;
   le sel de 2-[naphto(1,2-d)-thiazol-2-yl]-3-(4-nitrophényl)-5-[4-(2',3'-dihydroxypropylthio)phényl}-tétrazolium;
   le sel de 2-[naphto(1,2-d)-thiazol-2-yl]-3-(4-carboxyphényl]-5-(4-acétamidophényl)tétrazolium;
   le sel de 2-[naphto(1,2-d)-thiazol-2-yl]-3-(4-carboxyphényl)-5-[4-(2-(2-(2-hydroxyéthoxy)éthoxy)-éthoxy)phényl]tétrazolium;
   le sel de 2-[naphto(1,2-d)-thiazol-2-yl]-3-(2-méthyl-4-carboxyphényl)-5-(3,4-méthylènedioxyphényl)tétrazolium;
   le sel de 2-[naphto(1,2-d)-thiazol-2-yl]-3-(2-méthyl-4-nitrophényl)-5-(3,4-méthylènedioxyphényl)tétrazolium; et
   le sel de 2-[naphto(1,2-d)-thiazol-2-yl]-3-(4-cyanophényl)-5-(3-méthoxyphényl)tétrazolium, dans un milieu que l'on suspecte de contenir la substance réductrice et de déterminer la présence de la substance réductrice par un changement de couleur dans le sel de tétrazolium.

2. Utilisation des composés spécifiés à la revendication 1 pour la détection d'une substance réductrice, de préférence NADH.

3. Réactif pour la détection d'une substance réductrice, comprenant un ou plusieurs des composés selon la revendication 1.

4. Bandelette d'essai pour la détection d'une substance réductrice comprenant un ou plusieurs des composés selon la revendication 1.

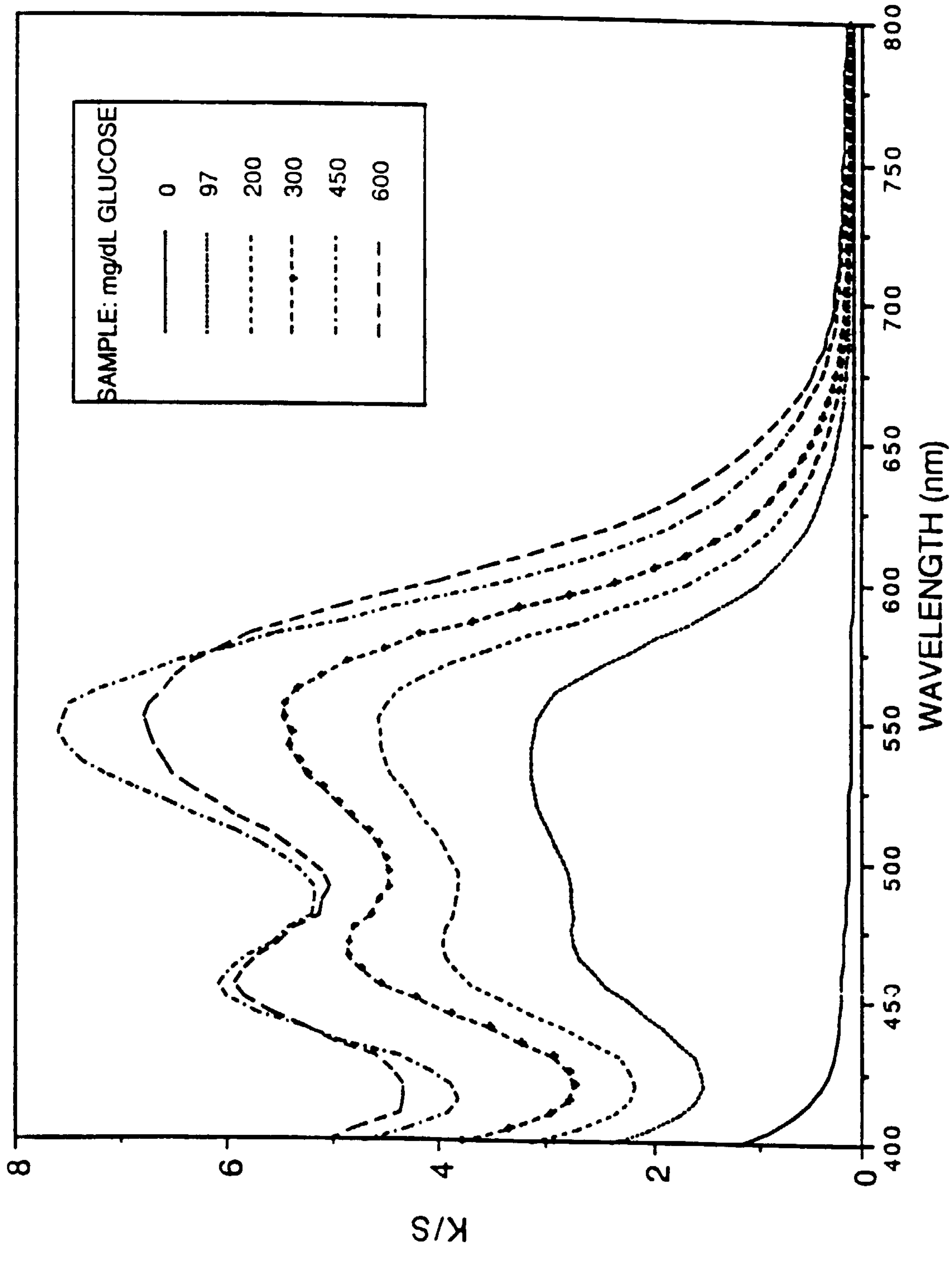
SAMPLE: mg/dL GLUCOSE
0
97
200
300
450
600
K/S
0
2
4
6
8
WAVELENGTH (nm)
400
450
500
550
600
650
700
750
800

FIG. 1

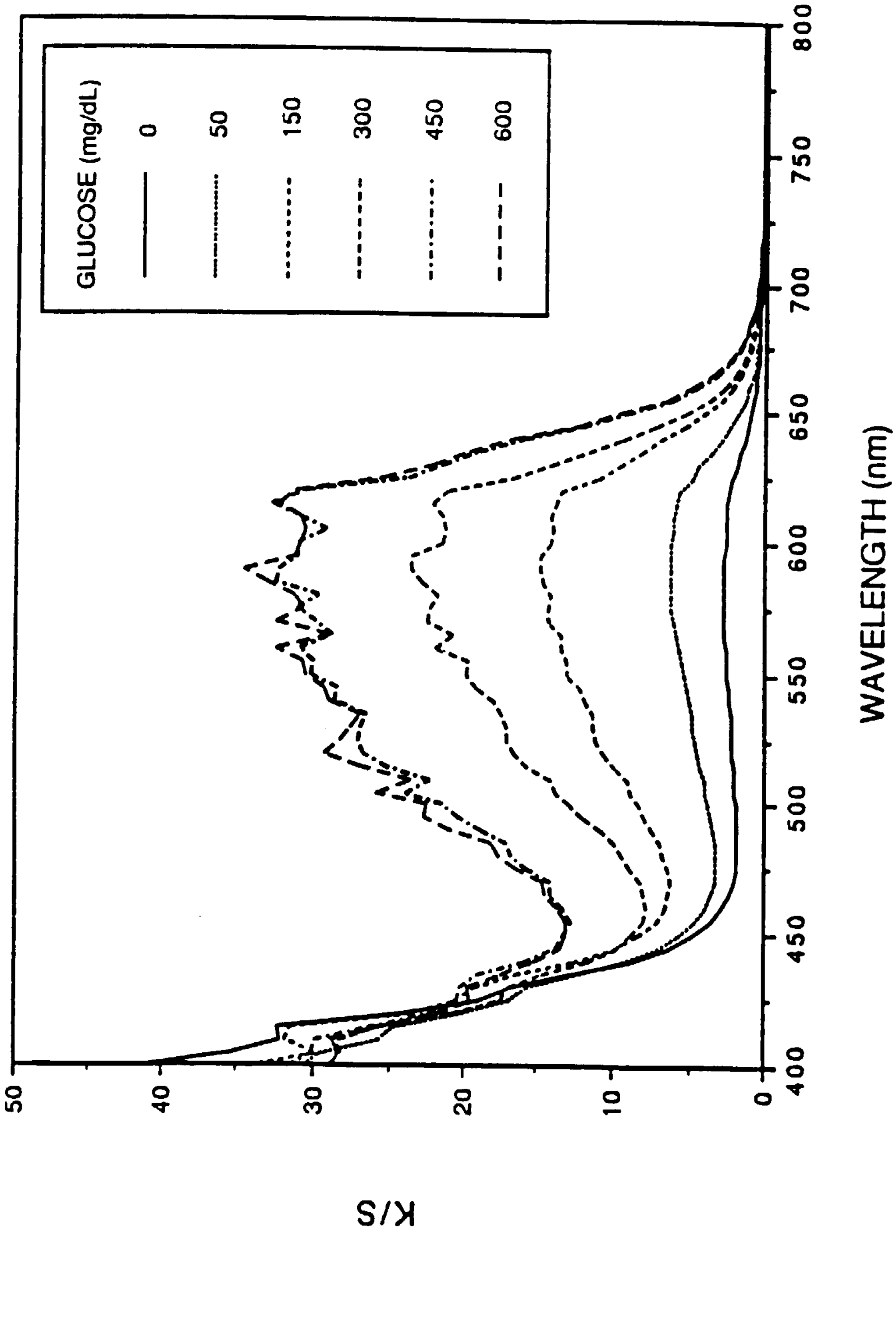
GLUCOSE (mg/dL)
0
50
150
300
450
600
K/S
0
10
20
30
40
50
400
450
500
550
600
650
700
750
800
WAVELENGTH (nm)

FIG. 2

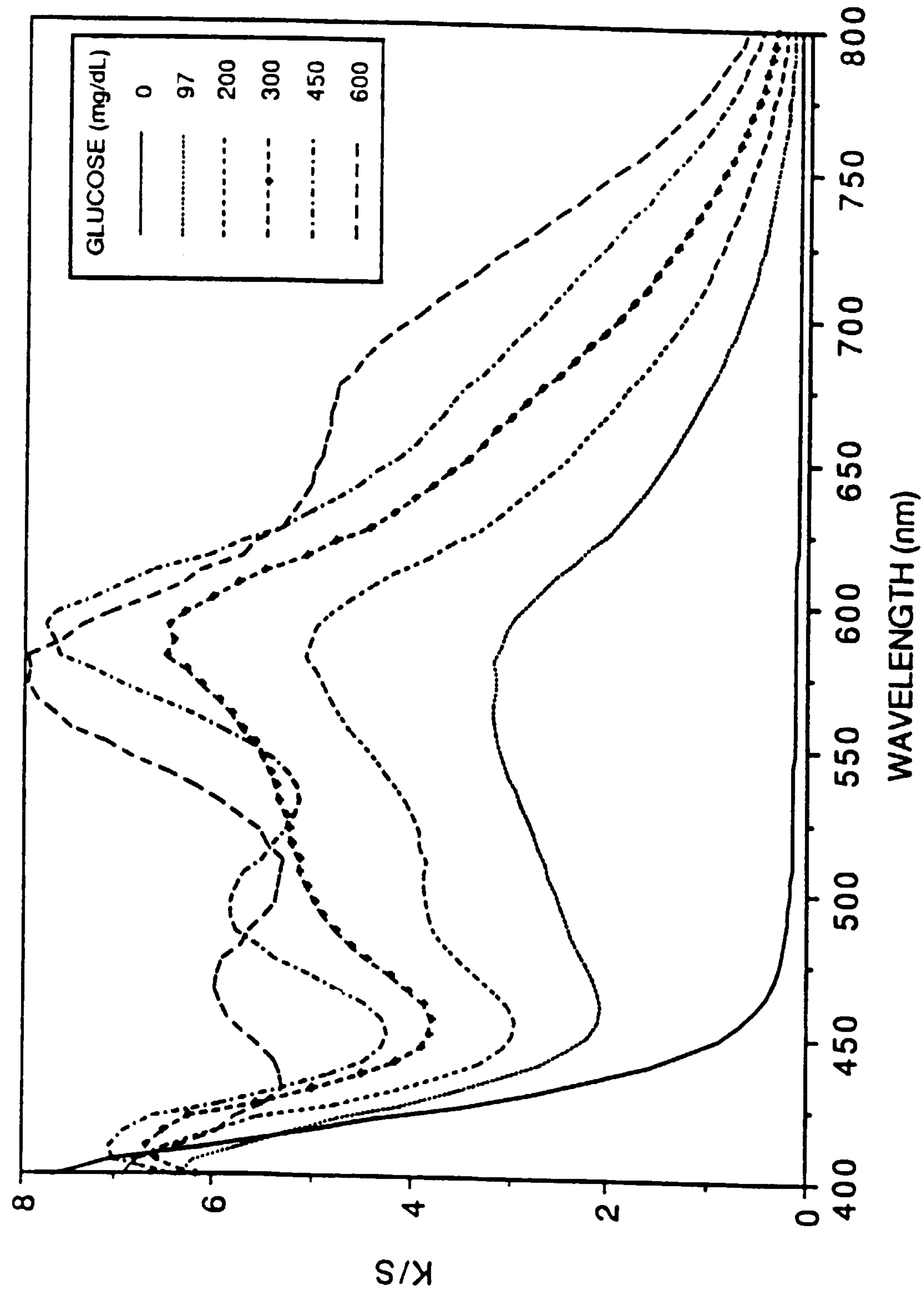
GLUCOSE (mg/dL)
0
97
200
300
450
600
K/S
0
2
4
6
8
400
450
500
550
600
650
700
750
800
WAVELENGTH (nm)

FIG. 3

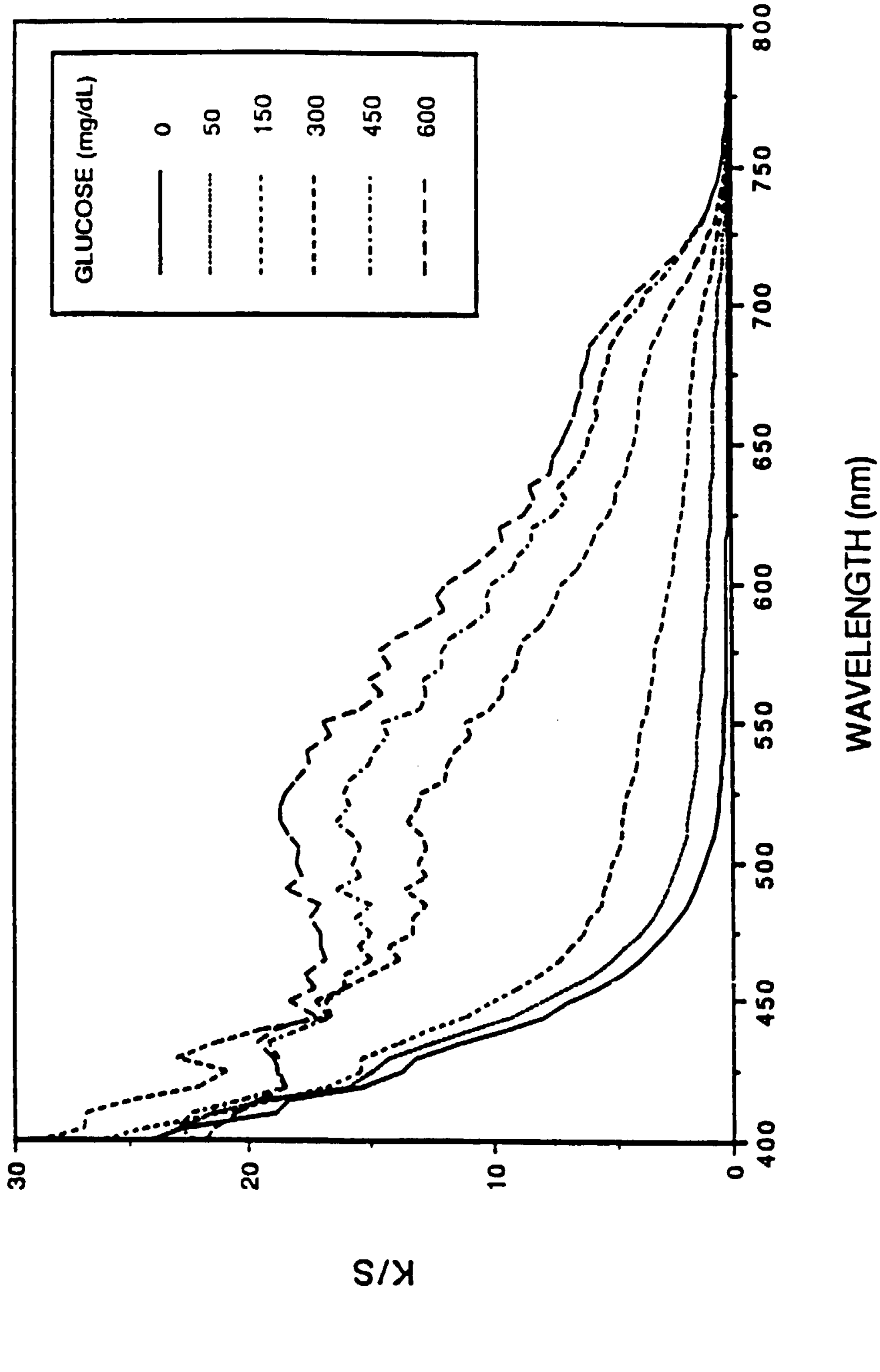
GLUCOSE (mg/dL)
0
50
150
300
450
600
K/S
30
20
10
0
400
450
500
550
600
650
700
750
800
WAVELENGTH (nm)

FIG. 4

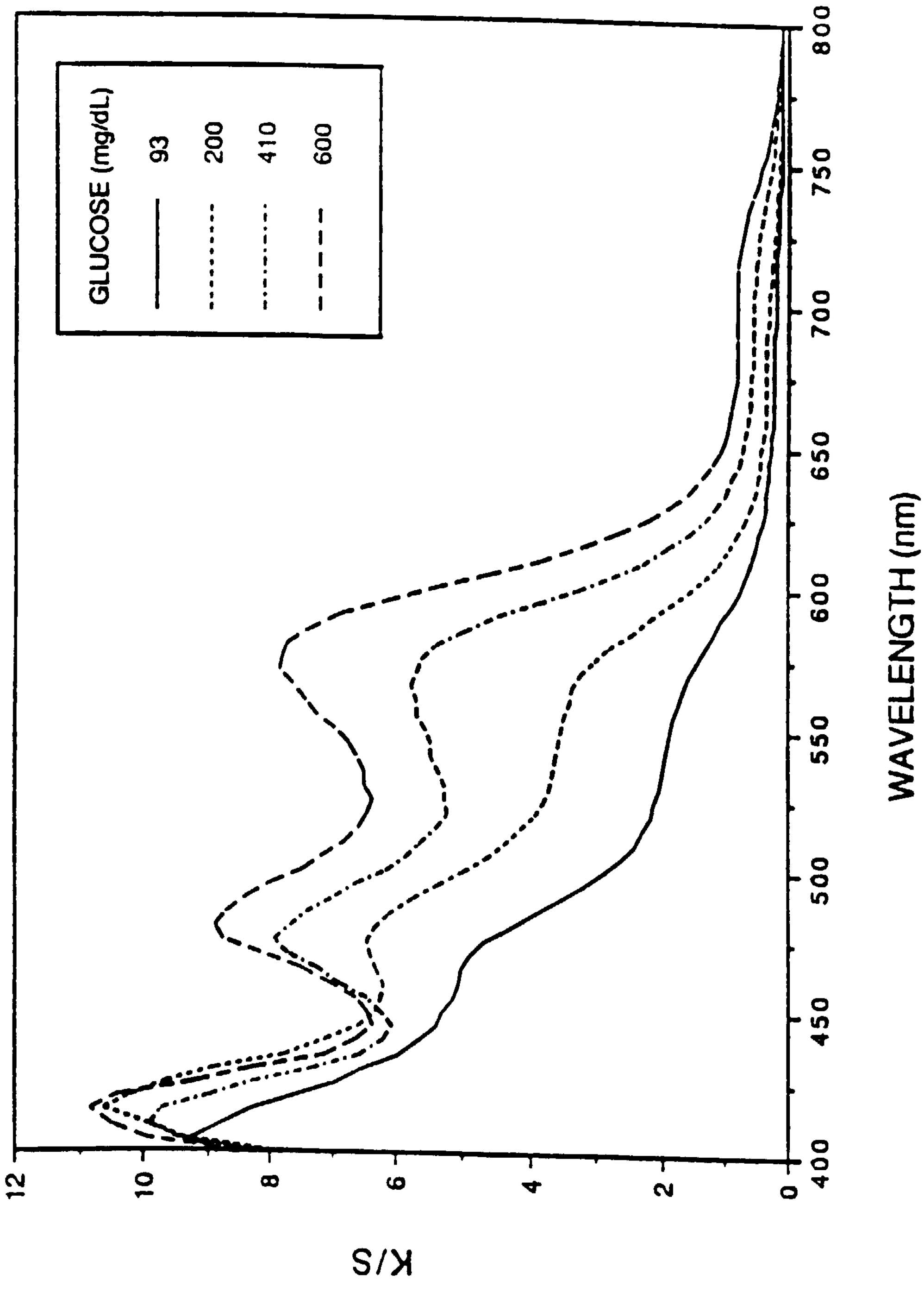
GLUCOSE (mg/dL)
93
200
410
600
K/S
0
2
4
6
8
10
12
400
450
500
550
600
650
700
750
800
WAVELENGTH (nm)

FIG. 5

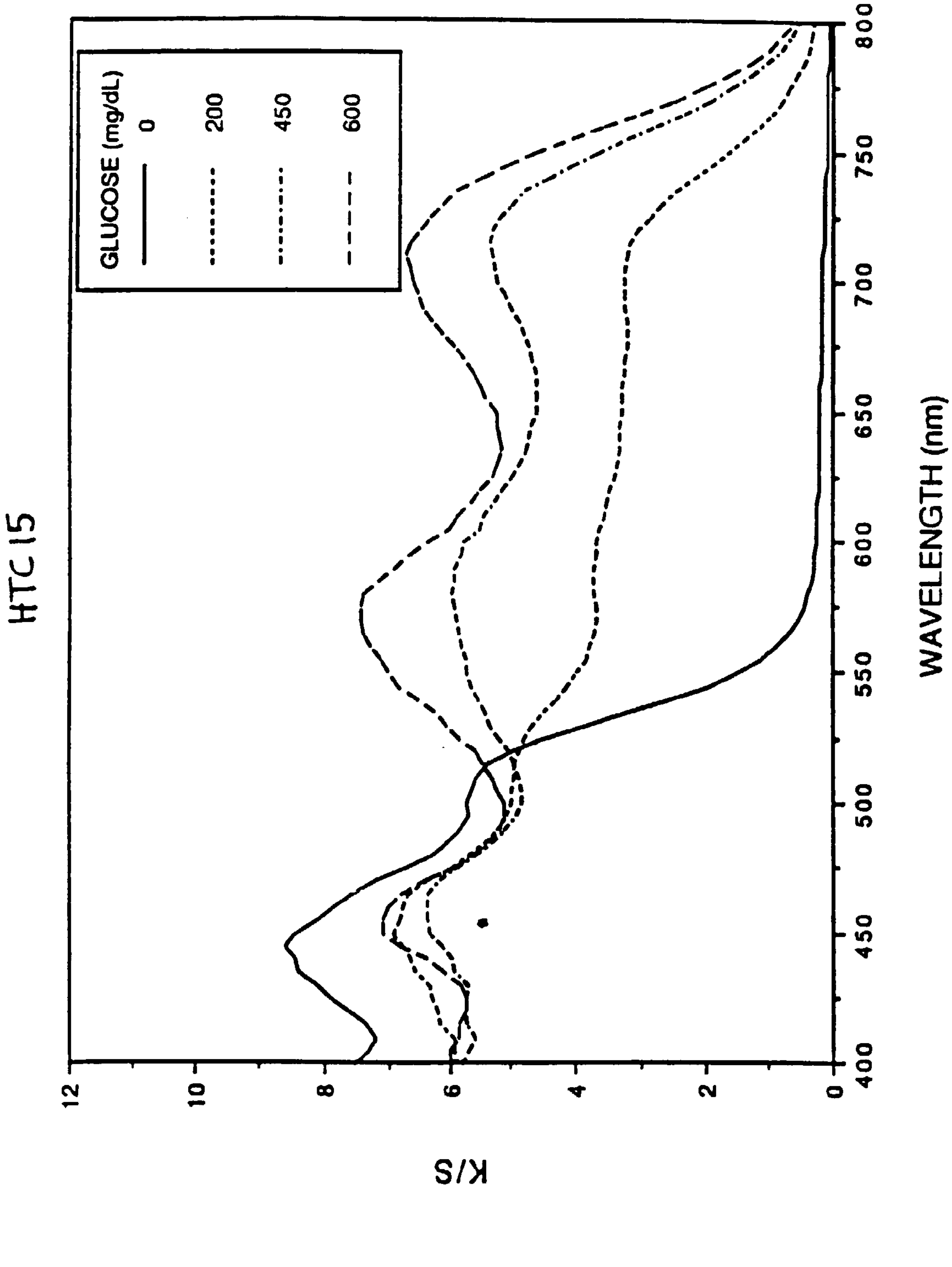
HTC 15
K/S
0
2
4
6
8
10
12
400
450
500
550
600
650
700
750
800
WAVELENGTH (nm)
GLUCOSE (mg/dL)
0
200
450
600

FIG. 6

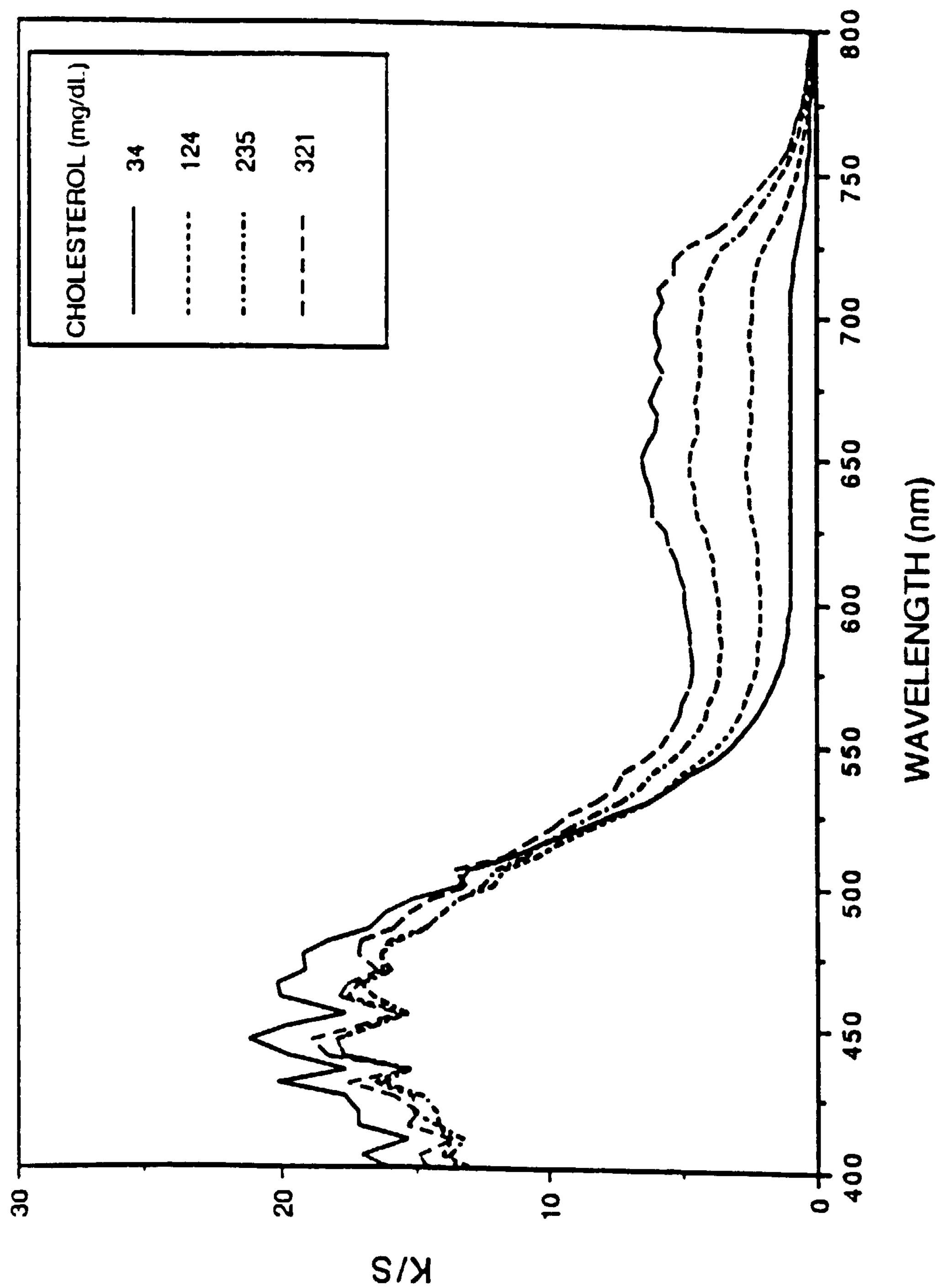
CHOLESTEROL (mg/dl.)
34
124
235
321
K/S
30
20
10
0
400
450
500
550
600
650
700
750
800
WAVELENGTH (nm)

FIG. 7

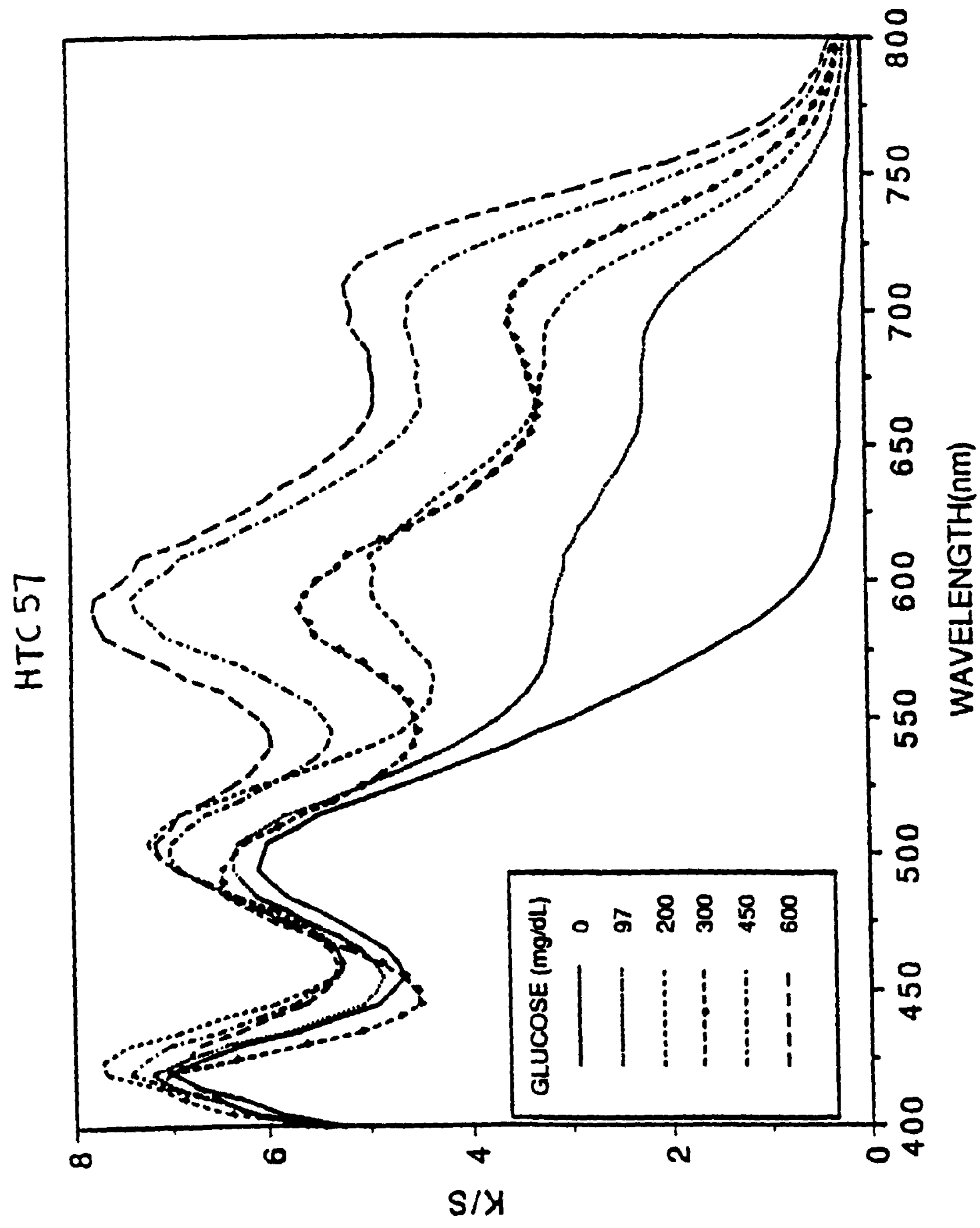
HTC 57
K/S
0
2
4
6
8
400
450
500
550
600
650
700
750
800
WAVELENGTH(nm)
GLUCOSE (mg/dL)
0
97
200
300
450
600

FIG. 8

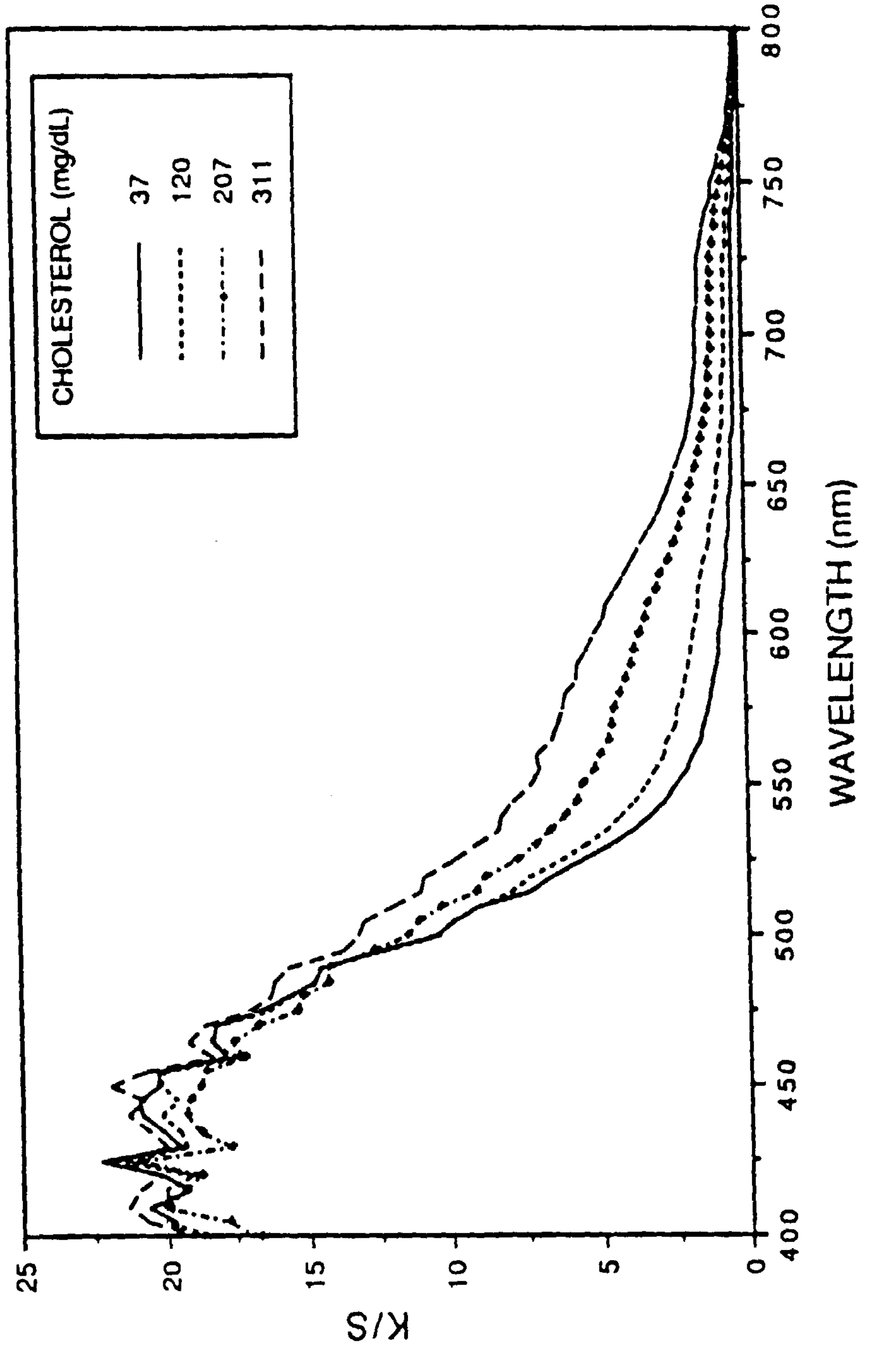
CHOLESTEROL (mg/dL)
37
120
207
311
K/S
25
20
15
10
5
0
400
450
500
550
600
650
700
750
800
WAVELENGTH (nm)

FIG. 9

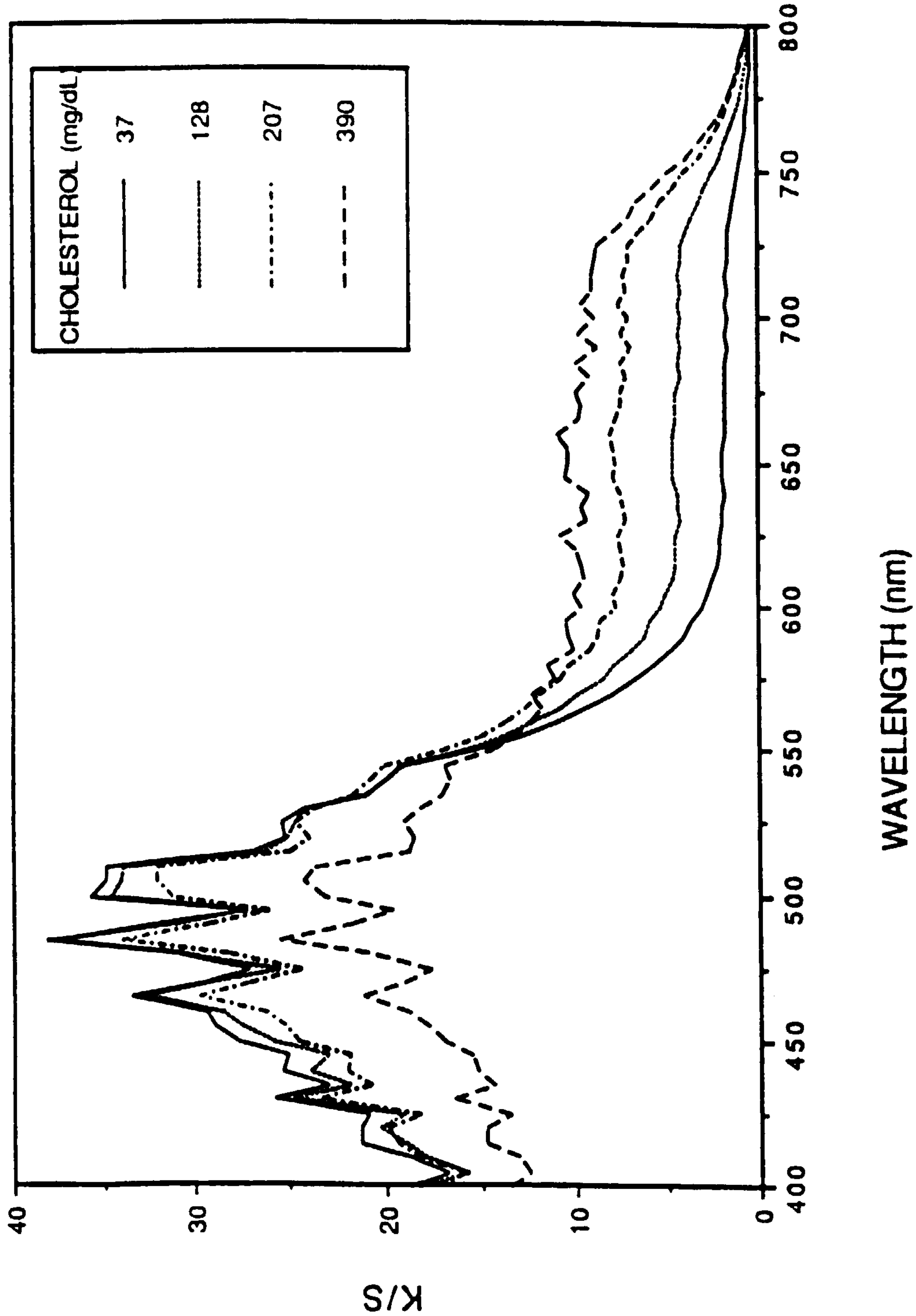
CHOLESTEROL (mg/dL)
37
128
207
390
K/S
0
10
20
30
40
400
450
500
550
600
650
700
750
800
WAVELENGTH (nm)

FIG. 10